# EUROPEAN PATENT APPLICATION

(11) **EP 4 647 503 A1**
(43) Date of publication of application: **12.11.2025**
(21) Application number: 24738634.5
(22) Date of filing: 04.01.2024
(51) Int. Cl.: C12Q 1/02, C12N 5/10, C12Q 1/66, C07K 14/62, C12N 15/17

(54) **METHOD FOR QUANTIFYING INSULIN SECRETION CAPACITY IN HUMAN CELL**

(30) Priority: 04.01.2023 JP 2023000268
(71) Applicant: Juntendo Educational Foundation, Tokyo 113-8421 (JP)
(72) Inventor: ENDOH, Hideki, Tokyo 113-8421 (JP); MATSUMOTO, Masahito, Tokyo 113-8421 (JP); OKAZAKI, Yasushi, Tokyo 113-8421 (JP)
(74) Representative: Plougmann Vingtoft a/s
(86) International application number: PCT/JP2024/000009
(87) International publication number: WO 2024/147344

(57) **Abstract**

Provided is an approach which can rapidly and inexpensively quantify human insulin secreted from human cells. The present invention provides a method for quantifying insulin secretion capacity in a human cell, comprising culturing a recombinant cell and measuring an amount of a secreted fused C-peptide, the recombinant cell being prepared by replacing an insulin gene in the genome of a human cultured cell line with a gene which expresses a fused C-peptide having a detectable tag peptide inserted in a C-peptide chain in a human proinsulin molecule.

## Description

### Technical Field

The present invention relates to a method for quantifying insulin secretion capacity in a human cell and use thereof.

### Background Art

Type I diabetes mellitus is a disease which causes substantial loss of insulin from cells (β cells) which produce and secrete insulin in the pancreas. The number of patients in Japan is approximately 1,100,000 people, and the number of overseas patients is approximately 1,100,000 people. 78,000 people per year develop the disease, which exhibits a fast-growing tendency.

Insulin injection, a current treatment method, is symptomatic treatment and causes hypoglycemia leading to coma or is difficult in blood glucose control. Burdens of blood glucose control influence QOL of patients' families. Due to the scarcity of brain-dead donors, only a limited number of patients are eligible for islet transplantation, and only adults are eligible.

In this respect, regenerative transplantation treatment of human β cells is desired, and regenerative medicine which exploits iPS cells has received attention. However, such treatment with iPS cells has problems such as the risk of tumorigenicity and has not yet reached a clinical stage. Meanwhile, the present inventors reported a technique of inducing the differentiation of somatic cells into β cells by direct reprogramming (Patent Literatures 1 to 3). However, the technique has not yet achieved sufficient induction efficiency of differentiation, particularly, for human cells.

Even in the case of using β cells obtained by direct reprogramming, it is important to develop a component that promotes insulin secretion capacity. The screening of such a component that promotes insulin secretion requires development of a screening system thereof. Techniques of detecting a proliferation marker or proliferation signals of pancreatic islet cells using human pancreatic islet cells have been reported as approaches therefor (Non Patent Literatures 1 and 2). A screening system using replication-controlling activity in rat β cells as an indicator has also been reported (Non Patent Literature 3). A technique of detecting the insulin secretion capacity in rat and mouse cells by replacing a C-peptide moiety of a proinsulin molecule with luciferase has been further reported (Patent Literature 4).

### Citation List

### Patent Literature

Patent Literature 1: WO 2016/002937
Patent Literature 2: WO 2017/073740
Patent Literature 3: WO 2021/095811
Patent Literature 4: U.S. Patent Application Publication No. 2020017485

### Non Patent Literature

Non Patent Literature 1: J Biomol Screen. 2012 Apr; 17 (4): 509-518
Non Patent Literature 2: Am J Physiol Endocrinol Metab. 2016 Nov 1; 311 (5): 859-E868
Non Patent Literature 3: Endocrinology. 2018 Sep; 159 (9): 3143-3157

### Summary of Invention

### Technical Problem

However, in the methods described in Non Patent Literatures 1 and 2, it is difficult to obtain human pancreatic islet cells to be used, and the methods are directed to detecting a molecule which influences only proliferation, and cannot screen for a component having an effect on whole β cell functions including insulin secretion capacity. The method described in Non Patent Literature 3 is directed to detecting replicating activity of rat β cells and cannot screen for a component having an effect on whole β cell functions including insulin secretion capacity. In the method described in Patent Literature 1, C-peptide of mouse insulin is fused with a long structure of luciferase, and the resulting fusion product cannot accurately reflect response to human insulin. Furthermore, a large size of the luciferase molecule might partially impair secretion capacity. Thus, it is difficult to reflect complete insulin secretion.

Thus, the present invention is directed to providing an approach which can rapidly and inexpensively quantify the human insulin secreted from human cells.

### Solution to Problem

Accordingly, the present inventors found that insulin secretion capacity can be rapidly and inexpensively quantified in human cells by using a gene which expresses a fused C-peptide prepared by identifying a position which does not inhibit insulin expression and secretion in a C-peptide moiety in a proinsulin molecule and inserting a highly sensitively detectable short-chain peptide at the position, to thereby complete the present invention.

Specifically, the present invention provides the following items [1] to [17] of the invention.
[1] A method for quantifying insulin secretion capacity in a human cell, comprising culturing a recombinant cell and measuring an amount of a secreted fused C-peptide, the recombinant cell being prepared by replacing an insulin gene in the genome of a human cultured cell line with a gene which expresses a fused C-peptide having a detectable tag peptide inserted in a C-peptide chain in a human proinsulin molecule.
[2] The quantification method according to [1], wherein an insertion position of the detectable tag peptide is a position corresponding to the C-terminal side of 7V of the C-peptide chain.
[3] The quantification method according to [1] or [2], wherein the detectable tag peptide is a tag peptide selected from the group consisting of a tag peptide detectable by chemiluminescent assay, and a tag peptide detectable by immunoassay.
[4] The quantification method according to any of [1] to [3], wherein the detectable tag peptide is a tag peptide selected from the group consisting of a tag peptide capable of binding to luciferase, a DDDDK-containing tag peptide, and a hemagglutinin-derived tag peptide.
[5] The quantification method according to any of [1] to [4], wherein the detectable tag peptide is a tag peptide capable of binding to luciferase.
[6] The quantification method according to any of [1] to [5], wherein the detectable tag peptide is a tag peptide selected from the group consisting of VSGWRLFKKIS (SEQ ID NO: 1), YPYDVPDYA (SEQ ID NO: 51), and YPYDVPDYA (SEQ ID NO: 51).
[7] The quantification method according to any of [1] to [6], wherein the human cultured cell line is (1) a human cultured cell line, (2) a human cell having 3 genes Ngn3, Mafa, and Pdx1 introduced therein, or (3) a human cell having 3 genes Ngn3, Mafa, and Pdx1; KLF9, KLF11, or KLF12 gene; and UCN3 gene simultaneously introduced therein.
[8] A recombinant cell prepared by replacing an insulin gene in the genome of a human cultured cell line with a gene which expresses a fused C-peptide having a detectable tag peptide inserted in a C-peptide chain in a human proinsulin molecule.
[9] The recombinant cell according to [8], wherein an insertion position of the detectable tag peptide is a position corresponding to the C-terminal side of 7V of the C-peptide chain.
[10] The recombinant cell according to [8] or [9], wherein the detectable tag peptide is a tag peptide selected from the group consisting of a tag peptide detectable by chemiluminescent assay, and a tag peptide detectable by immunoassay.
[11] The recombinant cell according to any of [8] to [10], wherein the detectable tag peptide is a tag peptide selected from the group consisting of a tag peptide capable of binding to luciferase, a DDDDK-containing tag peptide, and a hemagglutinin-derived tag peptide.
[12] The recombinant cell according to any of [8] to [11], wherein the detectable tag peptide is a tag peptide capable of binding to luciferase.
[13] The recombinant cell according to any of [8] to [12], wherein the detectable tag peptide is a tag peptide selected from the group consisting of VSGWRLFKKIS (SEQ ID NO: 1), YPYDVPDYA (SEQ ID NO: 51), and YPYDVPDYA (SEQ ID NO: 51).
[14] The recombinant cell according to any of [8] to [13], wherein the human cultured cell line is (1) a human cultured cell line, (2) a human cell having 3 genes Ngn3, Mafa, and Pdx1 introduced therein, or (3) a human cell having 3 genes Ngn3, Mafa, and Pdx1; KLF9, KLF11, or KLF12 gene; and UCN3 gene simultaneously introduced therein.
[15] A method for screening for a substance that promotes human insulin production and secretion, comprising culturing the recombinant cell according to any of [8] to [14] in the presence of a test substance, and measuring an amount of a secreted fused C-peptide.
[16] A human insulin production and secretion promoter, comprising a substance obtained by the screening method according to [15].
[17] An insulin production and secretion promoter, comprising one or two members selected from the group consisting of valproic acid and zebularine.

### Advantageous Effects of Invention

According to the present invention, the human insulin secreted from human cells can be rapidly and inexpensively quantified. Thus, a component that has an effect on whole β cell functions including insulin secretion capacity can be rapidly and inexpensively screened for.

### Brief Description of Drawings

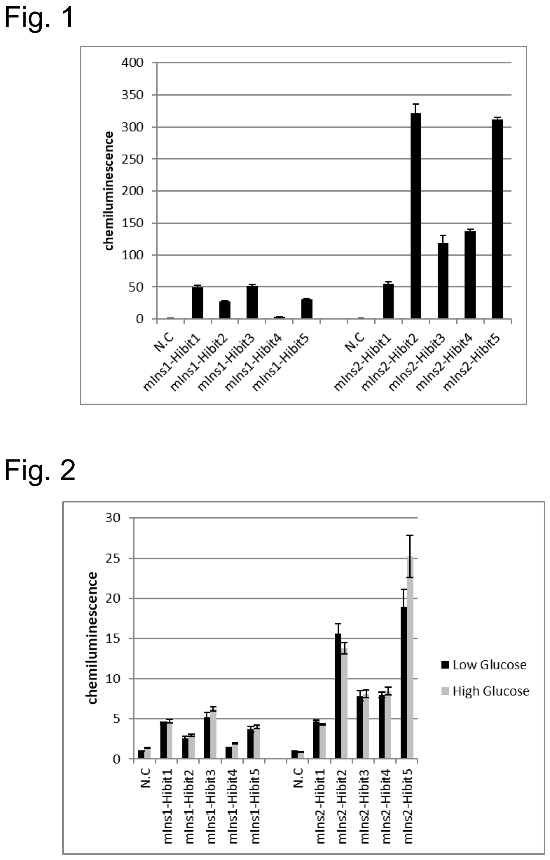

Figure 1 shows results of introducing each of mouse insulin 1-HiBiT and insulin 2-HiBiT fusion protein expression plasmids having 5 different types of structures into a mouse pancreatic β cell line MIN6 and then comparing expression-increasing effects on amounts of the insulin protein produced in the cells after culture for 3 days. The ordinate depicts amounts of the insulin-HiBiT fusion proteins contained in cell lysates, wherein the amounts were indicated in terms of amounts of chemiluminescence measured with luciferase activity as an indicator. The error bars each depict a standard deviation of measurement values of overlapped triple test samples.
Figure 2 shows amount of insulin-HiBiT fusion proteins extracellularly secreted in terms of amounts of chemiluminescence, which were determined by introducing each of mouse insulin 1-HiBiT and insulin 2-HiBiT fusion protein expression plasmids having 5 different types of structures into a mouse pancreatic β cell line MIN6, culturing the cell line for 3 days, and then measuring the amounts of chemiluminescence measured with luciferase activity as an indicator. The error bars each depict a standard deviation of measurement values of overlapped triple test samples.

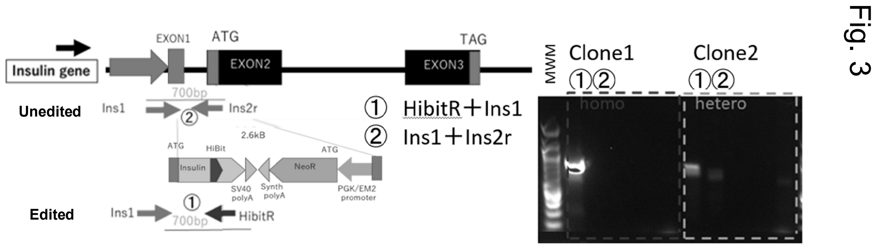

Figure 3 shows results of integrating a gene encoding a human insulin-HiBiT fusion protein into the insulin genome of a human adipose-derived cultured cell line SW872 by a genome editing method, performing single-cell separation with drug resistance as an indicator and expansion culture, and then confirming by genomic PCR and agarose electrophoresis whether genome editing occurred in one or both of the alleles.

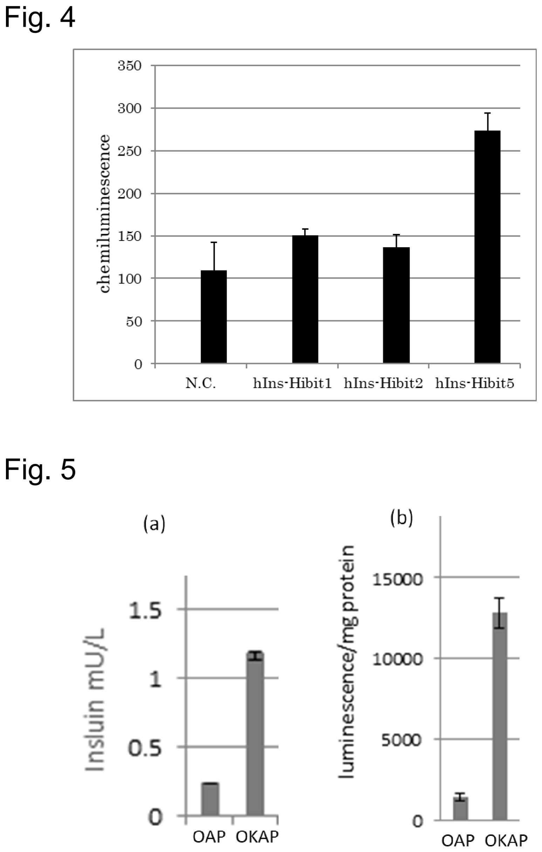

Figure 4 shows results of integrating a gene encoding each of human insulin-HiBiT fusion proteins having 3 different types of structures into the genome of a human fat-adipose cultured cell line SW872, culturing the cells so that 4 types of transcriptional factors (O: Ngn3, K: Glis1, A: MafA, and P: Pdx1) were co-expressed to induce direct reprogramming into β cells, and comparing amounts of the insulin protein consequently produced and secreted from the cells. The ordinate depicts amounts of the HiBiT protein contained in cell culture solutions, wherein the amounts were indicated in terms of amounts of chemiluminescence measured with luciferase activity as an indicator. The error bars each depict a standard deviation of measurement values of overlapped triple test samples.
Figure 5 shows results of integrating a human insulin-HiBiT fusion gene which maintained extracellular secretion capacity into one allele of the genome of a human adipose-derived cultured cell line SW872, culturing the cell line so as to co-express3 types of transcriptional factors (O: Ngn3, A: MafA, and P: Pdx1), then continuously culturing the cells under induction or non-induction conditions of a gene K factor that promotes direct reprogramming into β cells, and comparing amounts of the human insulin-HiBiT fusion protein produced from the cells. The ordinate of the graph (a) depicts amounts of the human insulin protein measured by ELISA, and the ordinate of the graph (b) shows luciferase activity from HiBiT. The error bars each depict a standard deviation of measurement values of overlapped triple test samples.

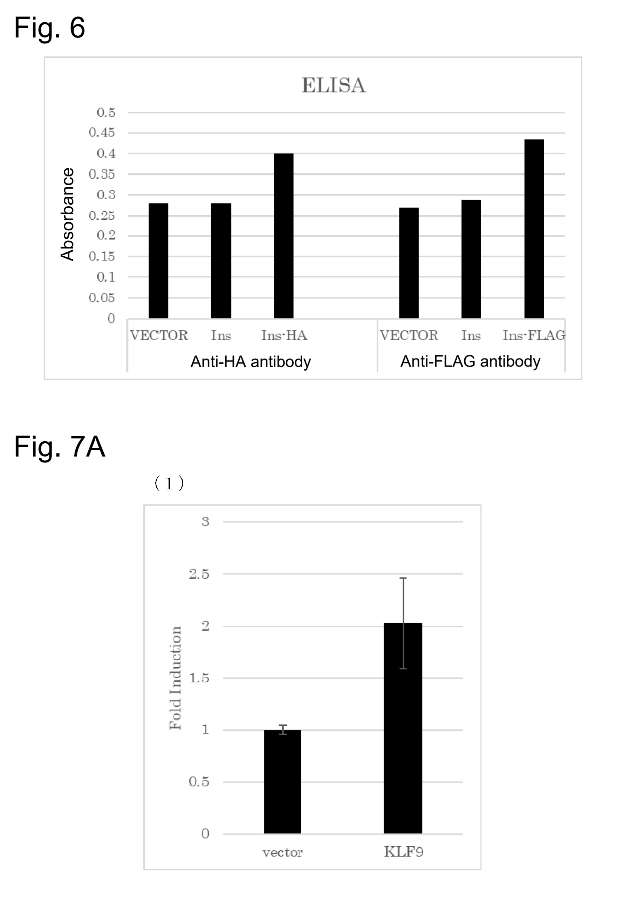

Figure 6 shows the detection, by ELISA, of a protein in culture supernatants of human SW872 cells into which only a plasmid, a human insulin expression plasmid, a human insulin-HA expression plasmid, and a human insulin-FLAG expression plasmid were introduced, respectively. Results obtained from both of a HA antibody and a FLAG antibody indicate that human insulin-HA and human insulin-FLAG were produced and secreted into the culture supernatants.
Figure 7A shows result of integrating a human insulin-HiBiT fusion gene which maintained extracellular secretion capacity into the genome of a human adipose-derived cultured cell line SW872, culturing the cells so as to co-express 3 types of transcriptional factors (Ngn3, MafA, and Pdx1), introducing an expression plasmid encoding each gene by transfection and comparing amounts of the human insulin-HiBiT fusion protein produced from the cells, to thereby search for a gene that promotes direct reprogramming into β cells. The ordinate depicts amounts of the HiBiT fusion protein contained in cell culture solutions, wherein the amounts were indicated in terms of amounts of chemiluminescence measured with luciferase activity as an indicator. The error bars each depict a standard deviation of measurement values of overlapped triple test samples. Figure 7A(1) shows that the introduction of a gene encoding human KLF9 increased the amount of the human insulin-HiBiT fusion protein produced.

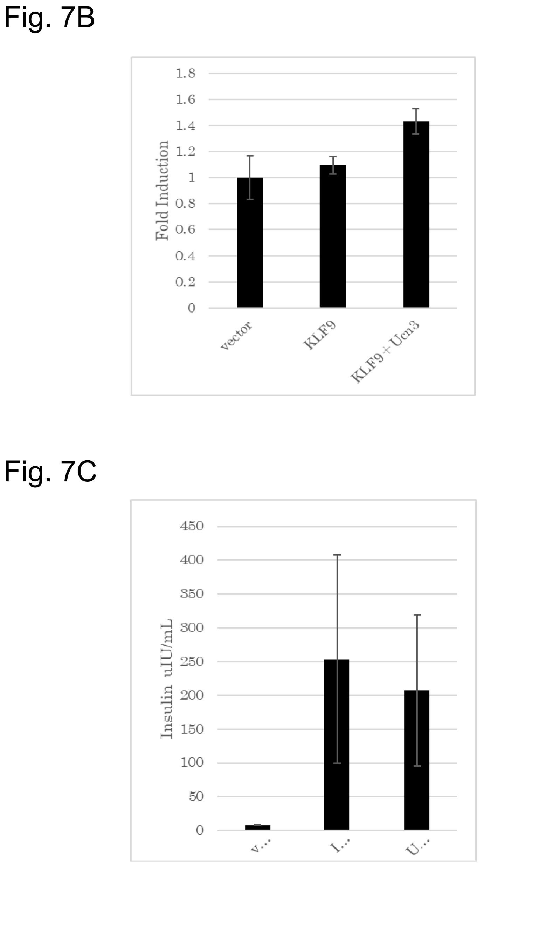

Figure 7B(2) shows that the simultaneous introduction of genes encoding human KLF9 and human UCN3 further increased the amount of the human insulin-HiBiT fusion protein produced.
Figure 7C(3) shows that a human adipose-derived cultured cell line Huh7 was cultured so as to co-express 3 types of transcriptional factors (Ngn3, MafA, and Pdx1), and transfected with an expression plasmid encoding insulin or KLF9 and UCN3 genes, and amounts of human insulin produced from the cells were compared. The ordinate depicts insulin contents in the cells. The error bars each depict a standard deviation of measurement values of overlapped triple test samples.

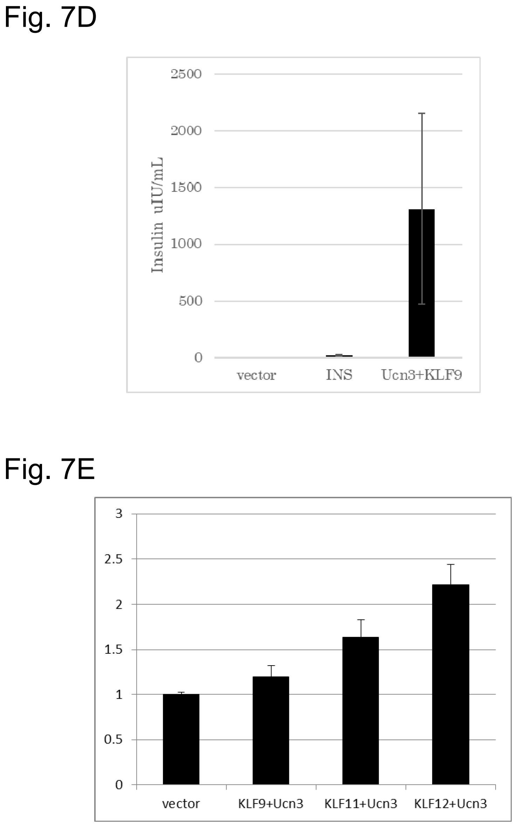

Figure 7D(4) shows results of culturing Huh7 so as to co-express 4 types of transcriptional factors (Ngn3, Glis1, MafA, and Pdx1), transfecting the cells with an expression plasmid encoding insulin or KLF9 and UCN3 genes, and comparing amounts of human insulin produced from the cells.
Figure 7E(5) shows results of culturing SW872 so as to co-express 4 types of transcriptional factors (Ngn3, Glis1, MafA, and Pdx1), co-transfecting the cells with expression plasmids encoding KLF9, KLF11, or KLF12 gene and UCN3 gene, and comparing amounts of human C-peptide produced from the cells. The error bars each depict a standard deviation of measurement values of overlapped triple test samples.

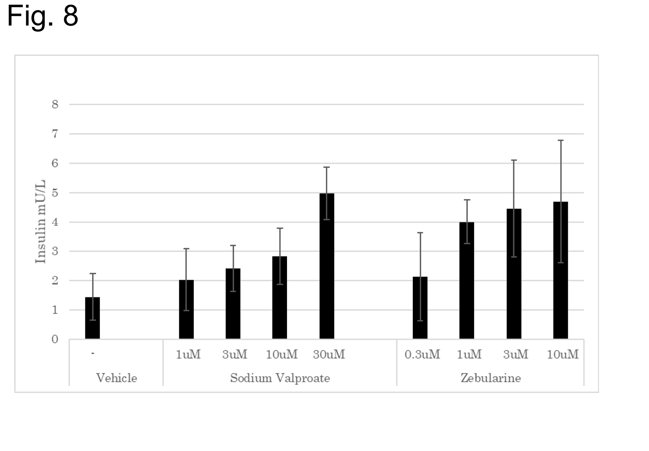

Figure 8 shows results of culturing a human adipose-derived cultured cell line SW872 having a human insulin-HiBiT fusion gene integrated into the genome to co-express 3 types of transcriptional factors (Ngn3, MafA, and Pdx1), adding each compound thereto, and comparing amounts of the human insulin-HiBiT fusion protein produced from the cells, to thereby search for a low-molecular compound that promotes direct reprogramming into β cells. The ordinate depicts amounts of the insulin-HiBiT fusion protein contained in cell lysates, wherein the amounts were indicated in terms of amounts of chemiluminescence measured with luciferase activity as an indicator. The error bars each depict a standard deviation of measurement values of overlapped triple test samples.

### Description of Embodiments

One aspect of the present invention provides a method for quantifying insulin secretion capacity in a human cell, comprising culturing a recombinant cell and measuring an amount of a secreted fused C-peptide, the recombinant cell being prepared by replacing an insulin gene in the genome of a human cultured cell line with a gene which expresses a fused C-peptide having a detectable tag peptide inserted in a C-peptide chain in a human proinsulin molecule.

Another aspect of the present invention provides a recombinant cell prepared by replacing an insulin gene in the genome of a human cultured cell line with a gene which expresses a fused C-peptide having a detectable tag peptide inserted in a C-peptide chain in a human proinsulin molecule.

A further alternative aspect of the present invention provides a method for screening for a substance that promotes human insulin production and secretion, comprising culturing the recombinant cell described above in the presence of a test substance and measuring an amount of a secreted fused C-peptide.

A further alternative aspect of the present invention provides a human insulin production and secretion promoter, comprising the substance obtained by the screening method described above.

A further alternative aspect of the present invention provides an insulin production and secretion promoter, comprising one or two members selected from the group consisting of valproic acid and zebularine.

As for insulin, its precursor proinsulin (having a structure where C-peptide is connected between insulin A chain and B chain) is produced by β cells and enzymatically degraded immediately before secretion to form one molecule each of insulin (having the A chain and the B chain linked together by a disulfide bond) and C-peptide. Thus, the amount of C-peptide in blood is a clinical test item for insulin secretion capacity, and ELISA, RIA, and RT-qPCR are known as its testing methods.

Human C-peptide is a peptide having 31 amino acids represented by EAEDLQVGQVELGGGPGAGSLQPLALEGSLQ (SEQ ID NO: 2).

It has not been entirely clear at what position a detectable tag peptide should be inserted in this C-peptide chain not to inhibit insulin expression and secretion. Accordingly, the present inventors first designed a plurality of fusion peptides having a detectable tag peptide inserted in mouse insulin C-peptide, and selected the structure of a fusion peptide which did not interfere with the production of mouse insulin. As a result, a fusion peptide having the tag inserted at two positions was confirmed not to inhibit insulin expression and secretion in mice. In this context, the insertion positions where insulin expression and secretion were not inhibited in mice were positions corresponding to 7V and 13G of human insulin C-peptide (SEQ ID NO: 2 described above).

Next, the present inventors designed a fusion peptide such that the detectable tag peptide was inserted at the same positions of human insulin C-peptide as the two positions of mouse insulin C-peptide, and studied for human insulin expression and secretion. As a result, it was found that use of a gene which expresses a fused C-peptide having the tag peptide inserted at a position corresponding to the C-terminal side of 7V of the human proinsulin C-peptide chain enables human insulin secretion capacity to be accurately measured without inhibiting human insulin expression and secretion.

The human insulin C-peptide does not have to have totally the same amino acids as the 31 amino acids and may have 1 to 3 amino acids on the C-terminal side and/or the N-terminal side and may lack 1 to 6 internal amino acids. The human insulin C-peptide may be, for example, RREAEDLQVGQVELGGGPGAGSLQPLALEGSLQKR (SEQ ID NO: 3), RREAEDLQVGGGPGAGSLQPLALEGSLQKR (SEQ ID NO: 4), EAEDLQVGGGPGAGSLQPLALEGSLQ (SEQ ID NO: 5), RREAEDLQVGQVELGGGPGAGSLQPLALEGSLQ (SEQ ID NO: 6), or EAEDLQVGQVELGGGPGAGSLQPLALEGSLQKR (SEQ ID NO: 7).

Examples of the detectable tag peptide used include a tag peptide selected from the group consisting of a tag peptide detectable by chemiluminescent assay, and a tag peptide detectable by immunoassay. In this context, examples of the tag peptide detectable by chemiluminescent assay include a tag peptide capable of binding to luciferase. Examples of the tag peptide detectable by immunoassay include a peptide selected from the group consisting of a DDDDK-containing tag peptide (FLAG tag) and a hemagglutinin-derived tag peptide (HA tag).

Further preferred examples of the detectable tag peptide include a tag peptide selected from the group consisting of VSGWRLFKKIS (SEQ ID NO: 1), YPYDVPDYA (SEQ ID NO: 51), and YPYDVPDYA SEQ ID NO: 51).

Among them, the detectable tag peptide is more preferably a tag peptide capable of binding to luciferase, further more preferably VSGWRLFKKIS (SEQ ID NO: 1), from the viewpoint of rapidly and inexpensively measuring the fused C-peptide.

In order to carry out the present invention, first, a gene which expresses a fused C-peptide having a detectable tag peptide inserted in a C-peptide chain in a human proinsulin molecule is constructed, and an insulin gene in the genome of a human cultured cell line is replaced with the obtained gene to obtain a recombinant cell. A usual genome editing approach, for example, ZFN, TALEN, or CRISPR/Cas9, can be used for replacing the insulin gene in the genome of a human cultured cell line with the gene which expresses the fusion peptide.

Examples of the human cultured cell line used include a liver-derived cell line Huh7 and a adipose-derived cell line SW872.

The present invention can also be adopted to a method for inducing a β cell having excellent insulin secretion capacity from a somatic cell by direct reprogramming. In this case, the cell used is preferably (1) a human cell having 3 genes Ngn3, Mafa, and Pdx1 introduced therein, or (2) a human cell having 3 genes Ngn3, Mafa, and Pdx1; KLF9, KLF11, or KLF12 gene; and UCN3 gene simultaneously introduced therein.

An amount of human insulin secreted in a human cell can be quantified by culturing the obtained recombinant cell and measuring an amount of a secreted fused C-peptide.

The recombinant cell can be cultured under usual conditions, for example, conditions involving a temperature of approximately 37°C and a CO₂ concentration of from 2 to 5%, using a culture medium suitable for the human cultured cell line, for example, DMEM, RPMI1640, MEM, αMEM, or M199.

In the case of using a tag peptide capable of binding to luciferase as the detectable tag peptide, the culture solution is reacted with a fragment of luciferase so that an amount of the fusion peptide secreted, i.e., an amount of human insulin secreted in the human cell, can be quantified from the amount of luminescence. In this context, commercially available HiBiT system (Promaga Corp.) can be employed for the fragment of luciferase used and the measurement of the amount of luminescence.

In the case of using a tag peptide detectable by immunoassay as the detectable tag peptide, an amount of a secreted fused C-peptide in the culture solution is estimated by immunoassay so that an amount of human insulin secreted in the human cell can be quantified. In this context, examples of the immunoassay include radioimmunoassay (RIA) and enzyme immunoassay (EIA). ELISA is particularly preferred.

A substance that promotes human insulin production and secretion can be screened for by culturing the recombinant cell described above in the presence of a test substance, culturing the recombinant cell described above and measuring an amount of a secreted fused C-peptide.

The amount of the secreted C-peptide is measured depending on the type of the tag peptide used, as described above.

A more preferable aspect provides a method for screening for a substance that promotes human insulin production and secretion, comprising culturing the recombinant cell in the presence of a test substance, allowing a fragment of luciferase to act thereon, and measuring insulin secretion capacity in the human cell.

In this context, the test substance may be a usual compound or may be a gene which influences the recombinant cell. The gene may be a gene introduced in advance into the recombinant cell.

When insulin production and secretion capacity is increased by the addition of the test compound or the gene, this test compound or gene can be determined to be a substance that promotes insulin production and secretion capacity of human β cells and is thus useful as an insulin production and secretion promoter.

In order to induce a β cell having excellent insulin secretion capacity from a somatic cell by direct reprogramming, an insulin secretion capacity-promoting gene obtained by the screening can be expressed, in addition to a gene described in Patent Literatures 1 to 3. In this context, examples of the gene described in Patent Literatures 1 to 3 include: Glis1 or Glis3 (K factor); 3 genes Ngn3Ngn3, Mafa and Pdx1; a combination of Glis1 or Glis3 with 3 genes Ngn3, Mafa and Pdx1; and 3 genes Ngn3, Mafa and Pdx1 and KLF9 and UCN3 genes to be simultaneously introduced into a human cell.

In this context, the somatic cell can be allowed to express these genes in accordance with an approach described in Patent Literatures 1 to 3. Specifically, a cultured somatic cell to be evaluated is coinfected with retrovirus vectors or lentivirus vectors into which the 3 genes described above have been respectively cloned. In a state in which these 3 genes or 5 genes are co-expressed in the cell, the cell can be cultured in a culture medium for differentiation induction described in Patent Literatures 1 to 3 for 1 to 2 weeks to obtain a β cell having insulin secretion capacity. Alternatively, the 3 genes or the 5 genes can be co-expressed in the cell using mRNA, oriP-EBNA1-based episomal vectors, Sendai virus vectors which are single-stranded RNA viruses, or the like. These methods can more safely produce differentiated β cells without integrating the transgenes into the host genome, as compared with the lentiviruses or the retroviruses mentioned above.

The substance obtained by the screening method is useful as a human insulin production and secretion promoter. Specifically, by this screening method, valproic acid and zebularine were found as components that promotes human insulin production and secretion. In short, one aspect of the present invention provides an insulin production and secretion promoter, comprising one or two members selected from the group consisting of valproic acid and zebularine.

Valproic acid has not been known to have an insulin secretion-promoting effect, though known as a therapeutic drug for epilepsy, mania, migraine, and the like. Zebularine, albeit being a DNA methylation inhibitor, has not been known to have an insulin secretion-promoting effect.

### Examples

Next, the present invention will be described in more detail with reference to Examples. However, the present invention is not limited by these Examples.

### Example 1

### (1) Design of mouse insulin C-peptide fusion peptide

As for mouse insulin protein, a precursor preproinsulin protein is processed to proinsulin by removal of a terminal signal sequence in the endoplasmic reticulum of pancreatic β cells, and C-peptide is cleaved therefrom to form insulin. Since equimolar amounts of the C-peptide and the insulin molecule are simultaneously formed and extracellularly secreted, insulin secretion capacity can be indirectly measured by the quantification of the C-peptide. Accordingly, HiBiT tag was inserted to a preferable site in a mouse proinsulin molecule to attempt the construction of a C-peptide-HiBiTHiBiT fusion peptide which enabled the fused HiBiT tag to be extracellularly secreted together with the C-peptide and rapidly and inexpensively quantified without impairing cleavage reaction of the C-peptide.

First, fusion proteins were designed such that HiBiT tag was inserted at five different positions in C-peptide of each of mouse insulin 1 and mouse insulin 2 or amino acids at the positions were substituted with the tag. The fusion proteins were examined for whether they influence cleavage and secretion of the C-peptide. Mere insertion of the HiBiT tag increases the molecular weight of a C-peptide fusion product and might influence its cleavage or secretion. Accordingly, amino acid sequences shown in SEQ ID NOs: 10, 12, 14, 16, and 18 were designed as HiBiT fusion proteins with the C-peptide region of mouse preproinsulin 1. These fusion proteins were each designed such that HiBiT was inserted between amino acids at positions 69 and 70 counted from the N terminus of mouse preproinsulin 1 or a peptide from position 71 to 78, or amino acids from position 62 to 68, from position 72 to 76, or from position 64 to 68 was replaced with HiBiT. Their encoding genes were cloned into expression plasmids.

These HiBiT fusion peptides with mouse preproinsulin 1 are referred to as mIns1-HiBiT1, mIns1-HiBiT2, mIns1-HiBiT3, mIns1-HiBiT4, and mIns1-HiBiT5 in order of description. The sequence of the expression plasmid encoding mIns1-HiBiT1 is shown in SEQ ID NO: 8. The nucleotide sequence of the gene encoding mIns1-HiBiT1 is shown in SEQ ID NO: 9, and the amino acid sequence thereof is shown in SEQ ID NO: 10. The mIns1-HiBiT2 to mIns1-HiBiT5 expression plasmids were prepared by replacing a nucleotide sequence moiety from position 1785 to 2141 of a sequence shown in SEQ ID NO: 8 with gene sequences shown in SEQ ID NOs: 11, 13, 15, and 17, respectively. All of these genes were synthesized by VectorBuilder Japan, Inc. (Tokyo, Japan) on consignment and purchased therefrom.

Likewise, amino acid sequences shown in SEQ ID NOs: 21, 23, 25, 27, and 29 were designed as HiBiT fusion proteins with the C-peptide region of mouse preproinsulin 2. These fusion proteins were each designed such that HiBiT was inserted between amino acids at positions 69 and 70 counted from the N terminus of mouse preproinsulin or a peptide from position 72 to 79, from position 62 to 68, from position 72 to 76, or from position 64 to 68 was replaced with HiBiT. Their encoding genes were cloned into expression plasmids. These HiBiT fusion peptides with mouse preproinsulin 2 are referred to as mIns2-HiBiT1, mIns2-HiBiT2, mIns2-HiBiT3, mIns2-HiBiT4, and mIns2-HiBiT5 in order of description. The sequence of the expression plasmid encoding mIns2-HiBiT1 is shown in SEQ ID NO: 19. The nucleotide sequence of the gene encoding mIns2-HiBiT1 is shown in SEQ ID NO: 20, and the amino acid sequence thereof is shown in SEQ ID NO: 21. The mIns2-HiBiT2 to mIns2-HiBiT5 expression plasmids were prepared by replacing a nucleotide sequence moiety from position 1786 to 2148 of a sequence shown in SEQ ID NO: 19 with gene sequences shown in SEQ ID NOs: 22, 24, 26, and 28, respectively. All of these genes were synthesized by VectorBuilder Japan, Inc. (Tokyo, Japan) on consignment and purchased therefrom.

### (2) Selection of structure which does not interfere with mouse insulin production

In order to examine whether the mouse insulin C-peptide fusion peptides designed in Example 1(1) influences intracellular gene expression and protein secretion, the expression vectors of these peptides were introduced into mouse pancreatic β cell line MIN6 cells (kindly provided by the University of Osaka) to examine expression behaviors. A frozen stock of MIN6 at passage number 21 was inoculated into a 10 cm culture dish and cultured in DMEM medium (Sigma D5796-500ML) containing 10% FBS and 1% antibiotic (Sigma penicillin-streptomycin) for 2 days. Then, the cells were liberated with 0.25 w/v% trypsin-1 mmol/l EDTA·4Na solution (containing phenol red) (WAKO 201-16945), and the number of cells was counted by using a hemocytometer, followed by inoculation at 4000 cells/well into a 96-well culture plate. On the next day, the culture medium was replaced with the above-mentioned one at 0.1 mL/well. Then, the cells in each cell-inoculated well of the 96-well plate described above were transformed with 0.16 µg in total of DNA composed of 0.08 µg each of 10 types in total of expression plasmids mIns1-HiBiT1 to mIns1-HiBiT5 and mIns2-HiBiT1 to mIns2-HiBiT5 supplemented with 0.08 µg of vector pGL3-luc (Promega Corp.) for normalization using Lipofectamine 2000 (Thermo Fisher Scientific Inc.) in accordance with the protocol. The culture was continued, and medium replacement was carried out 7 hours later. The cells were further cultured, and a glucose responsiveness test was conducted 2 days later.

The cells of each well were suspended in 100 µL of 1.4 mM glucose/KRB buffer (130 mM NaCl, 5 mM KCl, 1.2 mM CaCl₂, and 1.2 mM MgCl₂) and cultured at 37°C for 1 hour. Then, the cells are suspended in 100 µL of 2.8 mM glucose/KRB buffer and cultured for 1 hour. The buffer was removed, followed by sampling (Low glucose sample). The cells were subsequently suspended in 0.1 mL of 16.8 mM glucose/KRB buffer and cultured for 1 hour. The culture medium was removed in the same manner as above, followed by sampling (Hi glucose sample). The remaining cells were washed with 100 µL of ice-cold PBS(-), and the cells were then homogenized by the addition of 50 µL of x1 Cell Lysis buffer (Promega E194A) and pipetting, and recovered (cell lysate sample). Respective insulin contents in the samples were compared using HiBiT assay kit (Promega Corp., Nano-Glo HiBiT Extracellular Detection System).

As a result, as shown in Figure 1, no marked production of the insulin-HiBiT fusion proteins was observed for any of mIns1-HiBiT1 to mIns1-HiBiT5. By contrast, marked production of the HiBiT fusion proteins with mouse insulin 2 structurally similar to human insulin was observed for mIns2-HiBiT2 to mIns2-HiBiT5, and both expression and secretion of the fusion proteins were marked, particularly, for mIns2-HiBiT2 and mIns2-HiBiT5. Particularly, glucose-responsive secretion in mIns2-HiBiTHiBiT5 was also observed, suggesting that this fusion protein had the most preferable structure which did not interfere with the original properties of C-peptide.

### (3) Design of human insulin C-peptide fusion peptide

The results of Example 1(2) suggested that among the HiBiT fusion products with mouse insulin 2 structurally similar to human insulin, mIns2-HiBiT2 and mIns2-HiBiT5 had a structure which did not inhibit insulin expression and secretion. Accordingly, fusion proteins of human insulin and HiBiT were designed with reference to these structures. hIns-HiBiT1 homologous to mIns2-HiBiT1 as a negative control and hIns-HiBiT2 and hIns-HiBiT5 homologous to mIns2-HiBiT2 and mIns2-HiBiT5 as promising structures were designed. Their amino acid sequences are shown in SEQ ID NOs: 31, 33, and 35.

### (4) Introduction to genome of human cell line

DNAs (shown in SEQ ID NOs: 30, 32, and 34) encoding the 3 types of human insulin-HiBiT fusion proteins hIns-HiBiT1, hIns-HiBiT2, and hIns-HiBiT5 designed in Example 1(3) were synthesized by VectorBuilder Japan, Inc. (Tokyo, Japan) on consignment and purchased therefrom. The gene sequences encoding these 3 types of human insulin-HiBiT fusion proteins are shown in SEQ ID NOs: 30, 32, and 34.

A plasmid group for integrating these gene into an insulin gene in the genome of a human cell line for replacement by a genome editing technique was prepared.

First, Pre-Donor-arm1-HiBiT-arm2 plasmid shown in SEQ ID NO: 36 was synthesized by VectorBuilder Japan, Inc. (Tokyo, Japan) on consignment and purchased therefrom. PCR reaction was performed with this plasmid as a template using primers consisting of sequences shown in SEQ ID NOs: 37 and 38 to obtain a single-stranded DNA fragment. The PCR was carried out with a thermal cycler using PrimeSTAR(TM) Max DNA Polymerase (Takara Bio Inc.) and involved leaving a reaction solution at 98°C for 2 minutes, and repeating 25 cycles of continuous reactions of 98°C for 10 seconds, 55°C for 10 seconds, and 72°C for 30 seconds, followed by reaction at 72°C for 2 minutes. In parallel with this operation, PCR reaction was performed in the same manner as above with pcDNA13.1(-) (Thermo Fisher Scientific Inc.) as a template using primers shown in SEQ ID NOs: 39 and 40. The obtained two single-stranded DNAs were each purified by agarose electrophoresis and mixed at a ratio of 1:1. Then, Pre-Donor-arm1-NeoR-arm2, a plasmid shown in SEQ ID NO: 41, was prepared through In-Fusion ligation reaction using In-Fusion ligase (Takara Bio Inc.). PCR reaction was further performed in the same manner as above with this plasmid as a template using primers shown in SEQ ID NOs: 42 and 43 to obtain a single-stranded DNA fragment. In parallel with this operation, PCR was performed in the same manner as above with the hIns-HiBiT1-, hIns-HiBiT2-, or hIns-HiBiT5-encoding gene shown in SEQ ID NO: 30, 32, or 34 as a template using primers shown in SEQ ID NOs: 44 and 45 to obtain each single-stranded DNA fragment containing the hIns-HiBiT1-, hIns-HiBiT2-, or hIns-HiBiT5-encoding gene. Each of these single-stranded DNA fragments was mixed with the single-stranded DNA fragment obtained by PCR from the plasmid Pre-Donor-arm1-NeoR-arm2. Donor vectors for genome editing having the hIns-HiBiT1-, hIns-HiBiT2-, or hIns-HiBiT5-encoding gene, neomycin resistance gene, and arm sequences homologous to the insulin gene were constructed by In-Fusion ligation in the same manner as above. The sequence of Arm1-hIns-HiBiT1-NeoR-Arm2 containing hIns-HiBiT1 is shown as one of the donor vectors in SEQ ID NO: 46. The donor vector containing hIns-HiBiT2 or hIns-HiBiT5 was prepared by replacing a nucleotide sequence from position 60 to 432 of the Arm1-hIns-HiBiT1-NeoR-Arm2 sequence of SEQ ID NO: 46 with the sequence of hIns-HiBiT2 or hIns-HiBiT5 shown in SEQ ID NO: 32 or 34.

In order to integrate each sequence of hIns-HiBiT1, hIns-HiBiT2, or hIns-HiBiT5 in the donor vector into the genome at each moiety of human cells by genome editing, Guide RNA-hCAS9 plasmid shown in SEQ ID NO: 47, which carried guideRNA homologous to the insulin gene and hCAS9, was synthesized by VectorBuilder Japan, Inc. on consignment and purchased therefrom.

Cells of each of a human adipose-derived cell line SW872 (ATCC HTB-92(TM)) and a human liver-derived cell line Huh7 (kindly provided by the University of Osaka) were transfected with each of the Arm1-hIns-HiBiT1 to HiBiT5-NeoR-Arm2 donor vectors and the Guide RNA-hCAS9 vector at a ratio of 1:1 using Lipofectamine 3000. DMEM was used as a culture medium for Huh7, and Gibco DMEM/F12 (1:1) (cat# 11330-032) containing 10% FBS and 1% P/S was used as a culture medium for SW872. 7 hours after transfection, the culture medium was replaced while 400 µM resistance marker G418 was added to attempt the isolation of cells which underwent genome editing. Medium replacement was performed once per 3 days, and the cells were dissociated with trypsin/EDTA described above approximately 1 week later and subcultured to attempt single-cell separation using 96 wells. As a result, a single clone of cells presumably proliferated from one cell was obtained for both the Huh7 and SW872 cells in approximately 2 weeks, for each of hIns-HiBiT1, hIns-HiBiT2, and hIns-HiBiT5.

### (5) Confirmation of recombinant gene introduction

The genome-edited recombinant cell group obtained by proliferation from the single cells in Example 1(4) were subjected to genomic PCR to confirm whether the recombinant gene was actually introduced into the genome and whether genome editing occurred in one or both of the alleles. Each single clone of cells obtained by genome editing was inoculated into a 24-well culture plate and proliferated up to 100000 or more cells per well. Genomic DNA was extracted from each cell using Monarch Genomic DNA Purification Kit (New England Biolabs. Catalog No: T3010). PCR was performed with this DNA as a template using a primer set shown in SEQ ID NOs: 48 and 49 or a primer set shown in SEQ ID NOs: 48 and 50, and an amplified PCR band was confirmed by agarose gel electrophoresis to determine whether recombination occurred in one or both of the alleles. PCR reaction conditions were the same as those of the preceding section of this Example. Figure 3 shows the structures of the primers and the recombinant genes.

As a result, unedited alleles which yielded a band with the primer set of SEQ ID NOs: 48 and 49 and recombined cells which yielded a band with the primer set of SEQ ID NOs: 48 and 50 were almost equally found as shown in the gel electrophoretograms, demonstrating that both the homozygously and heterozygously edited cells were constructed.

### (6) Functional evaluation of human insulin-HiBiT fusion protein

Each of the recombinant proteins hIns-HiBiT1, hIns-HiBiT2, and hIns-HiBiT5 was functionally evaluated using the recombinant human cells that express the human insulin-HiBiT fusion proteins obtained in Example 1(5). The monoallelic recombinant SW872 cells obtained for each of the proteins were used, and the 3 types of recombinant cells were inoculated into 24-well culture dishes and proliferated. Almost confluent cells were obtained by culture for 3 days, and the culture medium was then replaced with a differentiation medium for β cells. The differentiation medium was prepared by adding 10 µM 2-mercaptoethanol, 10 µM ZnSO₄, 10 µM cAMP, and 10 mM nicotinamide (final concentrations) to the DMAM medium (Sigma D5796-500ML) containing 10% FBS and 1% antibiotic (Sigma penicillin-streptomycin) described above. In order to further cause direct reprogramming into β cells, the cell group was infected with Sendai virus vectors, which were single-stranded RNA viruses, carrying 4 genes Glis1, Ngn3, Mafa, and Pdx1, which were genes described in Patent Literatures 1 to 3 so that the cells were allowed to co-express the 4 genes. The Sendai virus was purchased from TOKIWA-Bio Inc.

As a result, only in the hIns-HiBiT5 recombinant protein, insulin expression from the cells was promoted, and insulin expression and secretion were able to be actually observed, as shown in Figure 4. The insertion position of HiBiT2 at which significant production and secretion were observed by fusion with mouse insulin 2 was not effective for humans, and only the hIns-HiBiT5 fusion product was found to have a structure which was able to be used in the quantification of insulin expression and secretion. In the drawing, N.C. denotes a negative control and depicts results obtained using recombinant SW872 cells having the hIns-HiBiT5 fusion product without being infected with the Sendai virus.

### Example 2

### (Screening)

Human recombinant cells which secrete the hIns-HiBiT5 fusion protein prepared in Example 1(5) and functionally tested in Example 1(6) serve as a powerful tool for comprehensively searching for genes, compounds, and other methods that promote differentiation into or functions of β cells, which have heretofore been difficult to be searched for due to expensive and time-consuming conventional quantification of insulin. Accordingly, the human recombinant cells prepared by the present invention were tested for whether they are actually able to be efficiently used in search for a novel gene that promotes β cell functions.

The human SW873 cells that produce hIns-HiBiT5 confirmed to have usefulness in Example 1(6) were infected with Sendai virus vectors that express 3 genes Ngn3, Mafa, and Pdx1 and cultured in a differentiation medium for β cells for 2 days. Then, Glis1, a gene described in Patent Literatures 1 to 3, carried by an episomal vector (FUJIFILM Wako Pure Chemical Corp.) was additionally introduced into the cells, which were then examined for differentiation into β cells, i.e., enhanced insulin expression. As a result, a significant insulin production-promoting effect was found by the additional administration of Glis1 carried by the episomal vector 2 days after infection with the Sendai virus. Here, in Figure 5(a), the amount of insulin was measured by use of ELISA (Mercodia), which requires a reaction time of 3 hours and a cost of 100 yen or higher per sample. The quantification of the HiBiT fusion protein shown in Figure 5(b) permitted measurement in a reaction time of 10 minutes at a cost of from 3 to 50 yen per sample.

### Example 3 Test on effectiveness of human insulin C-peptide fusion peptide using plural types of tags

The structure of hIns-HiBiT5 shown in Example 2 indicates a position at which a short peptide tag is inserted in an insulin molecule to thereby provide effective action. In order to show that the effectiveness of this peptide introduction position was not limited to the case of using the HiBiT peptide, it was confirmed that an amount of insulin produced and secreted was able to be measured even if the HiBiT peptide of hIns-HiBiT5 was replaced with other short peptides. Specifically, structures were prepared by replacement with HA tag (SEQ ID NO: 51) or FLAG tag (SEQ ID NO: 52) easily detectable with an antibody or the like, and examined for whether these tags were able to be detected without interfering with insulin production and secretion.

### (1) Preparation of hIns-HA and hIns-FLAG expression plasmids

PCR reaction was performed with cDNA (product No. HIcDNA133dT and HIcDNA-171dT, Primary Cell Co., Ltd.) of human pancreatic islet DNA (healthy donor) as a template using primers shown in SEQ ID NOs: 53 and 54 to obtain a single-stranded DNA fragment. The PCR was performed with a thermal cycler using PrimeSTAR(TM) Max DNA Polymerase (Takara Bio Inc.) and involved leaving a reaction solution at 98°C for 2 minutes, and repeating 25 cycles of continuous reactions of 98°C for 10 seconds, 55°C for 10 seconds, and 72°C for 30 seconds, followed by reaction at 72°C for 2 minutes. In parallel with this operation, an expression plasmid pEBMulti-Hyg (FUJIFILM Wako Pure Chemical Corp., product No. 050-08121, the number of bases: 11029 bp) was cleaved through reaction at 37°C for 1 hour by the addition of a restriction enzyme XhoI (New England Biolabs). The obtained two single-stranded DNAs were each purified by agarose electrophoresis and mixed at a ratio of 1:1. Then, an expression plasmid pEBMulti-hIns in which human insulin cDNA (complementary DNA: DNA consisting of a sequence from the start codon to the stop codon of a gene to be translated into a protein) shown in SEQ ID NO: 55 was cloned into the XhoI multicloning site of pEBMulti-Hyg was prepared through In-Fusion ligation reaction using In-Fusion ligase (Takara Bio Inc.). This plasmid expresses a human insulin precursor shown in SEQ ID NO: 56.

Subsequently, PCR reaction was performed with the pEBMulti-hIns thus prepared as a template using primers shown in SEQ ID NOs: 57 and 58 to obtain a single-stranded DNA fragment. PCR conditions are the same as above. PCR reaction was performed in the same manner as above with the pEBMulti-hIns as a template using primers shown in SEQ ID NOs: 61 and 62 to obtain a single-stranded DNA fragment. The obtained two single-stranded DNAs were each purified by agarose electrophoresis. Expression plasmids pEBMulti-hIns-HA and pEBMulti-hIns-FLAG in which hIns-HA shown in SEQ ID NO: 59 or hIns-FLAG shown in SEQ ID NO: 63 was cloned into the XhoI multicloning site of pEBMulti-Hyg were prepared through In-Fusion ligation reaction using In-Fusion ligase (Takara Bio Inc.).

### (2) Functional evaluation of human insulin-HA and -FLAG fusion proteins

SW872 cells inoculated into a 24-well culture dish were infected using the same differentiation medium and Sendai virus vectors, which were single-stranded RNA viruses, carrying 4 genes Glis1, Ngn3, Mafa, and Pdx1 (TOKIWA-Bio Inc.) as that of the preceding Example so that the cells were allowed to co-express the 4 genes. The culture medium was replaced with the differentiation medium for β cells mentioned above 4 hours later. After culture for 24 hours, the cells were transfected with each of the expression plasmids pEBMulti-hIns-HA and pEBMulti-hIns-FLAG obtained in the preceding section (1) and negative controls pEBMulti-Hyg and pEBMulti-hIns using Lipofectamine 3000. The culture medium was replaced 7 hours later, and the cells were continuously cultured for 2 days, followed by the recovery of 100 µL of a culture supernatant. The presence or absence of each fusion protein of HA or FLAG expressed and secreted from the cells in these culture supernatants was detected by ELISA using an antibody. First, an anti-HA antibody (HA-Tag Rabbit mAb (Cell Signaling Technology, Inc.)) or an anti-FLAG antibody (DYKDDDDK Tag Rabbit mAb (Cell Signaling Technology, Inc.)) was used as a primary antibody, diluted with a carbonic acid/hydrogen carbonate buffer solution (pH 9.8) so as to attain 100 ng/well, and immobilized on MaxiSorp 96-well plate (manufactured by NUNC A/S). After overnight reaction at 4°C, the plate was washed three times with TBS (Tris-buffered saline), and a TBS solution containing 3% bovine serum albumin (BSA) was added at 250 µL/well to each well. The plate was left standing at room temperature for 2 hours. The plate was washed three times with TBS, and each culture supernatant recovered above was added at 50 µL/well. The plate was left standing at room temperature for 1 hour.

After washing three times with TBS-T, a solution of Anti-rabbit IgG, HRP-linked Antibody (Cell Signaling Technology, Inc.) diluted 10000-fold with 1% BSA/TBS-T was added at 50 µL/well, and the plate was left standing at room temperature for 1 hour. Then, the plate was washed four times with TBS-T, and TMB Peroxidase Substrate (TMB Peroxidase EIA Substrate Kit, Bio-Rad Laboratories, Inc.) was added thereto. Then, the reaction was terminated with 1 mol/L phosphoric acid solution, followed by absorbance measurement at 450 nm using a spectrophotometry plate reader. As a result, as shown in Figure 6, clear signals were observed in the culture supernatant of the cells allowed to express hIns-HA or hIns-FLAG, as compared with the culture supernatant of the cells having only the negative control vector introduced therein or the cells allowed to express hIns, demonstrating that neither hIns-HA nor hIns-FLAG interfered with the production and secretion of the insulin fusion protein, as in hIns1-HiBiT5. Thus, the peptide insertion position of hIns1-HiBiT5 was found effective even if different peptide species were used. HiBiTHiBiT.

### Example 4 Identification of insulin production-promoting gene by screening

Example 2 above demonstrated that the human recombinant cells prepared by the present invention were actually able to be efficiently used in search for a novel gene that promotes β cell functions. Accordingly, a group of many human genes that may promote direct reprogramming into β cells was amplified by PCR from a human cDNA library by the same method as in Example 3(1), and cloned into a multicloning site of the expression plasmid pEBMulti-Hyg used in Example 3. Subsequently, human SW872 cells producing hIns-HiBiT5 confirmed to have usefulness in Example 1(6) were infected with Sendai virus vectors (TOKIWA-Bio Inc.) expressing 3 genes Ngn3, Mafa, and Pdx1, and cultured in a differentiation medium for β cells for 24 hours. These virus-infected cells were transfected with each human gene cloned into pEBMulti-Hyg using Lipofectamine 3000 and cultured in a differentiation medium for 5 days. Then, respective insulin contents in the samples were compared using HiBiT assay kit (Promega Corp., Nano-Glo HiBiT Extracellular Detection System).

As a result, as shown in Figure 7(1), enhanced production of the insulin-HiBiT fusion protein was observed by allowing recombinant SW872 cells that express 3 genes Ngn3, Mafa, and Pdx1 to co-express human KLF9 gene (SEQ ID NO: 71). The recombinant SW872 cells that express 3 genes Ngn3, Mafa, and Pdx1 used here were expanded by long-term culture, and the resulting cells were subjected to the same experiment as above. As a result, as shown in Figure 7(2), the co-expression of human KLF9 gene and human UCN3 gene (SEQ ID NO: 67) in combination was found to more markedly promote the production of the insulin-HiBiT fusion protein. Accordingly, a liver-derived cell line Huh7 was infected with Sendai virus vectors (TOKIWA-Bio Inc.) that express 3 genes Ngn3, Mafa, and Pdx1, cultured in a differentiation medium for β cells for 24 hours, and then transfected with expression plasmids of human KLF9 gene and human UCN3 gene using Lipofectamine 3000, and the amount of insulin produced was measured 5 days later. The expression plasmids of human KLF9 gene and human UCN3 gene were prepared by PCR and In-Fusion ligation reaction in the same manner as the method described in Example 3(1) using primers shown in SEQ ID NOs: 65 and 66 and SEQ ID NOs: 69 and 70. pEBMulti-Hyg was used as a negative control, and pEBMulti-hIns was used as a positive control. 12000 cells transfected with each plasmid were treated with acid ethanol (mixed solution of hydrochloric acid and ethanol), and a supernatant of the resulting cell extract was diluted 5-fold with a buffer, followed by the measurement of an insulin content. Insulin was quantified using HISCL HI-1000 (Sysmex Corp.). As shown in Figure 7(3), the human cells allowed to co-express human KLF9 gene and human UCN3 gene exhibited enhancement by 20 or more times in insulin production, as compared with the case of expressing only 3 genes Ngn3, Mafa, and Pdx1. In an experiment using the same conditions as above, as shown in Figure 7(4), more marked promotion of insulin production than that by the co-expression of human KLF9 gene and human UCN3 gene was caused when the Huh7 cells were infected with Sendai virus vectors (TOKIWA-Bio Inc.) that express 4 genes Ngn3, Mafa, and Pdx1 as well as Glis1. As seen from these results, the conventional method for direct reprogramming into human β cells plus the co-expression of KLF9 gene and human UCN3 gene acts to strongly promote human insulin production and secretion.

Subsequently, nucleotide sequence information on open reading frame was obtained for human KLF11 gene and KLF12 gene belonging to the same gene family as human KLF9 gene, with reference to accession Nos. NM_001198851.2 and NM_007249.5 of the reference sequence database of the National Center for Biotechnology Information. cDNA of each of these genes was cloned in the same manner as the method for cloning human KLF9 gene into the multicloning site of the expression plasmid pEBMulti-Hyg in the preceding section of this Example. Specifically, each expression plasmid was prepared by PCR and In-Fusion ligation reaction in the same manner as the method described in Example 3(1) using a primer having 30 bases from the start codon of each of the 3 genes linked to the 3' end of a nucleotide sequence CCTCACTAAAGGGGTACC, and a primer having a complementary strand sequence of 30 bases upstream from the stop codon linked to the 3' end of a nucleotide sequence AAGCTTATCGATACCGTC. A human adipose-derived cell line SW872 was infected with Sendai virus vectors (TOKIWA-Bio Inc.) that express 4 genes Ngn3, Mafa, Pdx1, and Glis1 in the same manner as above, then cultured in a differentiation medium for β cells for 24 hours, and then transfected with human KLF9 gene, human KLF11 gene, or human KLF11 gene together with the expression plasmid of human UCN3 gene using Lipofectamine 3000, and amounts of human C-peptide produced, which were proportional to amounts of insulin produced, in the culture solutions were measured 5 days later. pEBMulti-Hyg was used as a negative control. C-peptide was quantified using HISCL HI-1000 (Sysmex Corp.). As shown in Figure 7(5), the co-expression of human KLF11 gene or human KLF12 gene and human UCN3 gene was found to enhance the amount of C-peptide produced, as in human KLF9, as compared with the negative control. These results indicated that human KLF11 gene or human KLF12 gene, which was a gene belonging to the same family as the KLF9 gene, promoted the acquirement of human insulin production and secretion capacity by the conventional method for direct reprogramming into human β cells, when co-expressed with human UCN3 gene, as in the KLF9 gene.

### Example 5 Screening of low-molecular compound

Human SW872 cells that produce hIns-HiBiT5 were infected with Sendai virus vectors that express 3 genes Ngn3, Mafa, and Pdx1 in the same manner as in Example 4, and cultured in a differentiation medium for β cells for 24 hours. Each low-molecular compound was added thereto at a final concentration of from 0.3 to 30 nM, and the cells were cultured in a differentiation medium for 3 days and then further cultured for 2 days in a differentiation medium supplemented with the same compound at the same concentration as above. Respective insulin contents in the samples were compared using HiBiT assay kit to examine the presence or absence of a low-molecular compound that promotes differentiation into β cells. Compounds were screened using several types of commercially available compounds and a library of 9600 compounds provided by the Drug Discovery Initiative of the University of Tokyo. As a result, approximately 0.2% of compounds promoted the production of the insulin-HiBiT fusion protein. Among these compounds, two types were actually examined for an insulin production-promoting effect in Huh7 cells. As a result, as shown in Figure 8, valproic acid (Sigma-Aldrich Co., LLC., product No. P4543, CAS No. 1069-66-5) at a concentration of from 1 to 30 nM and zebularine (Tokyo Chemical Industry Co., Ltd., product No. Z0022, CAS No. 3690-10-6) at a concentration of from 0.3 to 10 nM promoted insulin production from the Huh7 cells. The method for quantifying insulin was performed in the same manner as in Example 4. Valproic acid was prepared into a 100 mM solution with ethanol, diluted, and added at a predetermined concentration to the cells. Zebularine was prepared into a 100 mM solution with dimethyl sulfoxide, diluted, and added to predetermined cells.

These results suggested that one or both of valproic acid and zebularine added in addition to the conventional method for direct reprogramming into human β cells can be utilized as a promoter for human insulin production and secretion from cells.

SEQ ID NO: 1
   HiBiT amino acid sequence
   VSGWRLFKKIS
SEQ ID NO: 2
   C-peptide-like sequence
   EAEDLQVGQVELGGGPGAGSLQPLALEGSLQ
SEQ ID NO: 3
   C-peptide-like sequence
   RREAEDLQVGQVELGGGPGAGSLQPLALEGSLQKR
SEQ ID NO: 4
   C-peptide-like sequence
   RREAEDLQVGGGPGAGSLQPLALEGSLQKR
SEQ ID NO: 5
   C-peptide-like sequence
   EAEDLQVGGGPGAGSLQPLALEGSLQ
SEQ ID NO: 6
   C-peptide-like sequence
   RREAEDLQVGQVELGGGPGAGSLQPLALEGSLQ
SEQ ID NO: 7
   C-peptide-like sequence
   EAEDLQVGQVELGGGPGAGSLQPLALEGSLQKR
SEQ ID NO: 8
   mIns1-HiBiT1 expression plasmid 
CAACTTTGTATAGAAAAGTTGCTCGACATTGATTATTGACTAGTTATTAATAGTAAT

CAATTACGGGGTCATTAGTTCATAGCCCATATATGGAGTTCCGCGTTACATAACTTA
 
CGGTAAATGGCCCGCCTGGCTGACCGCCCAACGACCCCCGCCCATTGACGTCAATAA
TGACGTATGTTCCCATAGTAACGCCAATAGGGACTTTCCATTGACGTCAATGGGTGG
AGTATTTACGGTAAACTGCCCACTTGGCAGTACATCAAGTGTATCATATGCCAAGTA
CGCCCCCTATTGACGTCAATGACGGTAAATGGCCCGCCTGGCATTATGCCCAGTACA
TGACCTTATGGGACTTTCCTACTTGGCAGTACATCTACGTATTAGTCATCGCTATTA
CCATGGTCGAGGTGAGCCCCACGTTCTGCTTCACTCTCCCCATCTCCCCCCCCTCCC
CACCCCCAATTTTGTATTTATTTATTTTTTAATTATTTTGTGCAGCGATGGGGGCGGG
GGGGGGGGGGGGGCGCGCGCCAGGCGGGGCGGGGCGGGGCGAGGGGCGGGGCGGGG
CGAGGCGGAGAGGTGCGGCGGCAGCCAATCAGAGCGGCGCGCTCCGAAAGTTTCCTT
TTATGGCGAGGCGGCGGCGGCGGCGGCCCTATAAAAAGCGAAGCGCGCGGCGGGCGG
GAGTCGCTGCGCGCTGCCTTCGCCCCGTGCCCCGCTCCGCCGCCGCCTCGCGCCGCC
CGCCCCGGCTCTGACTGACCGCGTTACTCCCACAGGTGAGCGGGCGGGACGGCCCTT
CTCCTCCGGGCTGTAATTAGCGCTTGGTTTAATGACGGCTTGTTTCTTTTCTGTGGC
TGCGTGAAAGCCTTGAGGGGCTCCGGGAGGGCCCTTTGTGCGGGGGGAGCGGCTCGG
GGGGTGCGTGCGTGTGTGTGTGCGTGGGGAGCGCCGCGTGCGGCTCCGCGCTGCCCG
GCGGCTGTGAGCGCTGCGGGCGCGGCGCGGGGCTTTGTGCGCTCCGCAGTGTGCGCG
AGGGGAGCGCGGCCGGGGGCGGTGCCCCGCGGTGCGGGGGGGGCTGCGAGGGGAACA
AAGGCTGCGTGCGGGGTGTGTGCGTGGGGGGGTGAGCAGGGGGTGTGGGCGCGTCGG
TCGGGCTGCAACCCCCCCTGCACCCCCCTCCCCGAGTTGCTGAGCACGGCCCGGCTT
CGGGTGCGGGGCTCCGTACGGGGCGTGGCGCGGGGCTCGCCGTGCCGGGCGGGGGGT
GGCGGCAGGTGGGGGTGCCGGGCGGGGCGGGGCCGCCTCGGGCCGGGGAGGGCTCGG
GGGAGGGGCGCGGCGGCCCCCGGAGCGCCGGCGGCTGTCGAGGCGCGGCGAGCCGCA
GCCATTGCCTTTTATGGTAATCGTGCGAGAGGGCGCAGGGACTTCCTTTGTCCCAAA
TCTGTGCGGAGCCGAAATCTGGGAGGCGCCGCCGCACCCCCTCTAGCGGGCGCGGGG
CGAAGCGGTGCGGCGCCGGCAGGAAGGAAATGGGCGGGGAGGGCCTTCGTGCGTCGC
CGCGCCGCCGTCCCCTTCTCCCTCTCCAGCCTCGGGGCTGTCCGCGGGGGGACGGCT
GCCTTCGGGGGGGACGGGGCAGGGCGGGGTTCGGCTTCTGGCGTGTGACCGGCGGCT
 
CTAGAGCCTCTGCTAACCATGTTCATGCCTTCTTCTTTTTCCTACAGCTCCTGGGCA
ACGTGCTGGTTATTGTGCTGTCTCATCATTTTGGCAAAGAATTGCAAGTTTGTACAA
AAAAGCAGGCTGCCACCATGGCCCTGTTGGTGCACTTCCTACCCCTGCTGGCCCTGC
TTGCCCTCTGGGAGCCCAAACCCACCCAGGCTTTTGTCAAACAGCATCTTTGTGGTC
CCCACCTGGTAGAGGCTCTCTACCTGGTGTGTGGGGAGCGTGGCTTCTTCTACACAC
CCAAGTCCCGCCGTGAAGTGGAGGACCCACAAGTGGAACAACTGGAGCTGGGAGTGA
GCGGCTGGCGGCTGTTCAAGAAGATTAGCGGAAGCCCCGGGGACCTTCAGACCTTGG
CGTTGGAGGTGGCCCGGCAGAAGCGTGGCATTGTGGATCAGTGCTGCACCAGCATCT
GCTCCCTCTACCAGCTGGAGAACTACTGCAACGGAAGCGGAGCCACGAACTTCTCTC
TGTTAAAGCAAGCAGGAGATGTTGAAGAAAACCCCGGGCCTATGGTGAGCAAGGGCG
AGGAGCTGTTCACCGGGGTGGTGCCCATCCTGGTCGAGCTGGACGGCGACGTAAACG
GCCACAAGTTCAGCGTGTCCGGCGAGGGCGAGGGCGATGCCACCTACGGCAAGCTGA
CCCTGAAGTTCATCTGCACCACCGGCAAGCTGCCCGTGCCCTGGCCCACCCTCGTGA
CCACCCTGACCTACGGCGTGCAGTGCTTCAGCCGCTACCCCGACCACATGAAGCAGC
ACGACTTCTTCAAGTCCGCCATGCCCGAAGGCTACGTCCAGGAGCGCACCATCTTCT
TCAAGGACGACGGCAACTACAAGACCCGCGCCGAGGTGAAGTTCGAGGGCGACACCC
TGGTGAACCGCATCGAGCTGAAGGGCATCGACTTCAAGGAGGACGGCAACATCCTGG
GGCACAAGCTGGAGTACAACTACAACAGCCACAACGTCTATATCATGGCCGACAAGC
AGAAGAACGGCATCAAGGTGAACTTCAAGATCCGCCACAACATCGAGGACGGCAGCG
TGCAGCTCGCCGACCACTACCAGCAGAACACCCCCATCGGCGACGGCCCCGTGCTGC
TGCCCGACAACCACTACCTGAGCACCCAGTCCGCCCTGAGCAAAGACCCCAACGAGA
AGCGCGATCACATGGTCCTGCTGGAGTTCGTGACCGCCGCCGGGATCACTCTCGGCA
TGGACGAGCTGTACAAGTAAACCCAGCTTTCTTGTACAAAGTGGTGATGGCCGGCCG
CTTCGAGCAGACATGATAAGATACATTGATGAGTTTGGACAAACCACAACTAGAATG
CAGTGAAAAAAATGCTTTATTTGTGAAATTTGTGATGCTATTGCTTTATTTGTAACC
ATTATAAGCTGCAATAAACAAGTTAACAACAACAATTGCATTCATTTTATGTTTCAG
GTTCAGGGGGAGGTGTGGGAGGTTTTTTAAAGCAAGTAAAACCTCTACAAATGTGGT
 
AGCGGCCGCGGCGCTCTTCCGCTTCCTCGCTCACTGACTCGCTGCGCTCGGTCGTTC
GGCTGCGGCGAGCGGTATCAGCTCACTCAAAGGCGGTAATACGGTTATCCACAGAAT
CAGGGGATAACGCAGGAAAGAACATGTGAGCAAAAGGCCAGCAAAAGGCCAGGAACC
GTAAAAAGGCCGCGTTGCTGGCGTTTTTCCATAGGCTCCGCCCCCCTGACGAGCATC
ACAAAAATCGACGCTCAAGTCAGAGGTGGCGAAACCCGACAGGACTATAAAGATACC
AGGCGTTTCCCCCTGGAAGCTCCCTCGTGCGCTCTCCTGTTCCGACCCTGCCGCTTA
CCGGATACCTGTCCGCCTTTCTCTCTTCGGGAAGCGTGGCGCTTTCTCATAGCTCAC
GCTGTAGGTATCTCAGTTCGGTGTAGGTCGTTCGCTCCAAGCTGGGCTGTGTGCACG
AACCCCCCGTTCAGCCCGACCGCTGCGCCTTATCCGGTAACTATCGTCTTGAGTCCA
ACCCGGTAAGACACGACTTATCGCCACTGGCAGCAGCCACTGGTAACAGGATTAGCA
GAGCGAGGTATGTAGGCGGTGCTACAGAGTTCTTGAAGTGGTGGCCTAACTACGGCT
ACACTAGAAGAACAGTATTTGGTATCTGCGCTCTGCTGAAGCCAGTTACCTTCGGAA
AAAGAGTTGGTAGCTCTTGATCCGGCAAACAAACCACCGCTGGTAGCGGTGGTTTTT
TTGTTTGCAAGCAGCAGATTACGCGCAGAAAAAAAGGATCTCAAGAAGATCCTTTGA
TCTTTTCTACGGGGTCTGACGCTCAGTGGAACGAAAACTCACGTTAAGGGATTTTGG
TCATGAGATTATCAAAAAGGATCTTCACCTAGATCCTTTTAAATTAAAAATGAAGTT
TTAAATCAATCTAAAGTATATATGAGTAAACTTGGTCTGACAGTTACCAATGCTTAA
TCAGTGAGGCACCTATCTCAGCGATCTGTCTATTTCGTTCATCCATAGTTGCCTGAC
TCCCCGTCGTGTAGATAACTACGATACGGGAGGGCTTACCATCTGGCCCCAGTGCTG
CAATGATACCGCGAGACCCACGCTCACCGGCTCCAGATTTATCAGCAATAAACCAGC
CAGCCGGAAGGGCCGAGCGCAGAAGTGGTCCTGCAACTTTATCCGCCTCCATCCAGT
CTATTAATTGTTGCCGGGAAGCTAGAGTAAGTAGTTCGCCAGTTAATAGTTTGCGCA
ACGTTGTTGCCATTGCTACAGGCATCGTGGTGTCACGCTCGTCGTTTGGTATGGCTT
CATTCAGCTCCGGTTCCCAACGATCAAGGCGAGTTACATGATCCCCCATGTTGTGCA
AAAAAGCGGTTAGCTCCTTCGGTCCTCCGATCGTTGTCAGAAGTAAGTTGGCCGCAG
TGTTATCACTCATGGTTATGGCAGCACTGCATAATTCTCTTACTGTCATGCCATCCG
TAAGATGCTTTTCTGTGACTGGTGAGTACTCAACCAAGTCATTCTGAGAATAGTGTA
 
TGCGGCGACCGAGTTGCTCTTGCCCGGCGTCAATACGGGATAATACCGCGCCACATA

GCAGAACTTTAAAAGTGCTCATCATTGGAAAACGTTCTTCGGGGCGAAAACTCTCAA

GGATCTTACCGCTGTTGAGATCCAGTTCGATGTAACCCACTCGTGCACCCAACTGAT

CTTCAGCATCTTTTACTTTCACCAGCGTTTCTGGGTGAGCAAAAACAGGAAGGCAAA

ATGCCGCAAAAAAGGGAATAAGGGCGACACGGAAATGTTGAATACTCATACTCTTCC

TTTTTCAATATTATTGAAGCATTTATCAGGGTTATTGTCTCATGAGCGGATACATAT

TTGAATGTATTTAGAAAAATAAACAAATAGGGGTTCCGCGCACATTTCCCCGAAAAG

TGCCACCTGACGTCTAAGAAACCATTATTATCATGACATTAACCTATAAAAATAGGC

GTATCACGAGGCCCTTTCGTCGGCGCGCCGCGGCCGC
SEQ ID NO: 9
   mIns1-HiBiT1-encoding gene 
ATGGCCCTGTTGGTGCACTTCCTACCCCTGCTGGCCCTGCTTGCCCTCTGGGAGCCC

AAACCCACCCAGGCTTTTGTCAAACAGCATCTTTGTGGTCCCCACCTGGTAGAGGCT

CTCTACCTGGTGTGTGGGGAGCGTGGCTTCTTCTACACACCCAAGTCCCGCCGTGAA

GTGGAGGACCCACAAGTGGAACAACTGGAGCTGGGAGTGAGCGGCTGGCGGCTGTTC

AAGAAGATTAGCGGAAGCCCCGGGGACCTTCAGACCTTGGCGTTGGAGGTGGCCCGG

CAGAAGCGTGGCATTGTGGATCAGTGCTGCACCAGCATCTGCTCCCTCTACCAGCTG

GAGAACTACTGCAAC
SEQ ID NO: 10
   mIns1-HiBiT1 amino acid sequence 
MALLVHFLPLLALLALWEPKPTQAFVKQHLCGPHLVEALYLVCGERGFFYTPKSRRE
VEDPQVEQLELGVSGWRLFKKISGSPGDLQTLALEVARQKRGIVDQCCTSICSLYQL
ENYCN
SEQ ID NO: 11
   mIns1-HiBiT2-encoding gene 
ATGGCCCTGTTGGTGCACTTCCTACCCCTGCTGGCCCTGCTTGCCCTCTGGGAGCCC

AAACCCACCCAGGCTTTTGTCAAACAGCATCTTTGTGGTCCCCACCTGGTAGAGGCT

CTCTACCTGGTGTGTGGGGAGCGTGGCTTCTTCTACACACCCAAGTCCCGCCGTGAA

GTGGAGGACCCACAAGTGGAACAACTGGAGCTGGGAGGAGTGAGCGGCTGGCGGCTG

TTCAAGAAGATTAGCGCGTTGGAGGTGGCCCGGCAGAAGCGTGGCATTGTGGATCAG

TGCTGCACCAGCATCTGCTCCCTCTACCAGCTGGAGAACTACTGCAAC
SEQ ID NO: 12
   mIns1-HiBiT2 amino acid sequence 
MALLVHFLPLLALLALWEPKPTQAFVKQHLCGPHLVEALYLVCGERGFFYTPKSRRE

VEDPQVEQLELGGVSGWRLFKKISALEVARQKRGIVDQCCTSICSLYQLENYCN
SEQ ID NO: 13
   mIns1-HiBiT3-encoding gene 
ATGGCCCTGTTGGTGCACTTCCTACCCCTGCTGGCCCTGCTTGCCCTCTGGGAGCCC
AAACCCACCCAGGCTTTTGTCAAACAGCATCTTTGTGGTCCCCACCTGGTAGAGGCT
CTCTACCTGGTGTGTGGGGAGCGTGGCTTCTTCTACACACCCAAGTCCCGCCGTGAA
GTGGAGGACCCAGTGAGCGGCTGGCGGCTGTTCAAGAAGATTAGCGGAGGAAGCCCC
GGGGACCTTCAGACCTTGGCGTTGGAGGTGGCCCGGCAGAAGCGTGGCATTGTGGAT
CAGTGCTGCACCAGCATCTGCTCCCTCTACCAGCTGGAGAACTACTGCAAC
SEQ ID NO: 14
   mIns1-HiBiT3 amino acid sequence 
MALLVHFLPLLALLALWEPKPTQAFVKQHLCGPHLVEALYLVCGERGFFYTPKSRRE
VEDPVSGWRLFKKISGGSPGDLQTLALEVARQKRGIVDQCCTSICSLYQLENYCN
SEQ ID NO: 15
   mIns1-HiBiT4-encoding gene 
ATGGCCCTGTTGGTGCACTTCCTACCCCTGCTGGCCCTGCTTGCCCTCTGGGAGCCC

AAACCCACCCAGGCTTTTGTCAAACAGCATCTTTGTGGTCCCCACCTGGTAGAGGCT

CTCTACCTGGTGTGTGGGGAGCGTGGCTTCTTCTACACACCCAAGTCCCGCCGTGAA

GTGGAGGACCCACAAGTGGAACAACTGGAGCTGGGAGGAAGCGTGAGCGGCTGGCGG

CTGTTCAAGAAGATTAGCACCTTGGCGTTGGAGGTGGCCCGGCAGAAGCGTGGCATT

GTGGATCAGTGCTGCACCAGCATCTGCTCCCTCTACCAGCTGGAGAACTACTGCAAC
SEQ ID NO: 16
   mIns1-HiBiT4 amino acid sequence 
MALLVHFLPLLALLALWEPKPTQAFVKQHLCGPHLVEALYLVCGERGFFYTPKSRRE

VEDPQVEQLELGGSVSGWRLFKKISTLALEVARQKRGIVDQCCTSICSLYQLENYCN
SEQ ID NO: 17
   mIns1-HiBiT5-encoding gene 
ATGGCCCTGTTGGTGCACTTCCTACCCCTGCTGGCCCTGCTTGCCCTCTGGGAGCCC

AAACCCACCCAGGCTTTTGTCAAACAGCATCTTTGTGGTCCCCACCTGGTAGAGGCT

CTCTACCTGGTGTGTGGGGAGCGTGGCTTCTTCTACACACCCAAGTCCCGCCGTGAA

GTGGAGGACCCACAAGTGGTGAGCGGCTGGCGGCTGTTCAAGAAGATTAGCGGAGGA

AGCCCCGGGGACCTTCAGACCTTGGCGTTGGAGGTGGCCCGGCAGAAGCGTGGCATT

GTGGATCAGTGCTGCACCAGCATCTGCTCCCTCTACCAGCTGGAGAACTACTGCAAC
SEQ ID NO: 18
   mIns1-HiBiT5 amino acid sequence 
MALLVHFLPLLALLALWEPKPTQAFVKQHLCGPHLVEALYLVCGERGFFYTPKSRRE

VEDPQVVSGWRLFKKISGGSPGDLQTLALEVARQKRGIVDQCCTSICSLYQLENYCN
SEQ ID NO: 19
   mIns2-HiBiT1 expression plasmid 
CAACTTTGTATAGAAAAGTTGCTCGACATTGATTATTGACTAGTTATTAATAGTAAT
CAATTACGGGGTCATTAGTTCATAGCCCATATATGGAGTTCCGCGTTACATAACTTA
CGGTAAATGGCCCGCCTGGCTGACCGCCCAACGACCCCCGCCCATTGACGTCAATAA
TGACGTATGTTCCCATAGTAACGCCAATAGGGACTTTCCATTGACGTCAATGGGTGG
AGTATTTACGGTAAACTGCCCACTTGGCAGTACATCAAGTGTATCATATGCCAAGTA
CGCCCCCTATTGACGTCAATGACGGTAAATGGCCCGCCTGGCATTATGCCCAGTACA
TGACCTTATGGGACTTTCCTACTTGGCAGTACATCTACGTATTAGTCATCGCTATTA
CCATGGTCGAGGTGAGCCCCACGTTCTGCTTCACTCTCCCCATCTCCCCCCCCTCCC
CACCCCCAATTTTGTATTTATTTATTTTTTAATTATTTTGTGCAGCGATGGGGGCGGG
GGGGGGGGGGGGGCGCGCGCCAGGCGGGGCGGGGCGGGGCGAGGGGCGGGGCGGGG
CGAGGCGGAGAGGTGCGGCGGCAGCCAATCAGAGCGGCGCGCTCCGAAAGTTTCCTT
TTATGGCGAGGCGGCGGCGGCGGCGGCCCTATAAAAAGCGAAGCGCGCGGCGGGCGG
GAGTCGCTGCGCGCTGCCTTCGCCCCGTGCCCCGCTCCGCCGCCGCCTCGCGCCGCC
CGCCCCGGCTCTGACTGACCGCGTTACTCCCACAGGTGAGCGGGCGGGACGGCCCTT
CTCCTCCGGGCTGTAATTAGCGCTTGGTTTAATGACGGCTTGTTTCTTTTCTGTGGC
TGCGTGAAAGCCTTGAGGGGCTCCGGGAGGGCCCTTTGTGCGGGGGGAGCGGCTCGG
GGGGTGCGTGCGTGTGTGTGTGCGTGGGGAGCGCCGCGTGCGGCTCCGCGCTGCCCG
GCGGCTGTGAGCGCTGCGGGCGCGGCGCGGGGCTTTGTGCGCTCCGCAGTGTGCGCG
AGGGGAGCGCGGCCGGGGGCGGTGCCCCGCGGTGCGGGGGGGGCTGCGAGGGGAACA
AAGGCTGCGTGCGGGGTGTGTGCGTGGGGGGGTGAGCAGGGGGTGTGGGCGCGTCGG
TCGGGCTGCAACCCCCCCTGCACCCCCCTCCCCGAGTTGCTGAGCACGGCCCGGCTT
CGGGTGCGGGGCTCCGTACGGGGCGTGGCGCGGGGCTCGCCGTGCCGGGCGGGGGGT
GGCGGCAGGTGGGGGTGCCGGGCGGGGCGGGGCCGCCTCGGGCCGGGGAGGGCTCGG
GGGAGGGGCGCGGCGGCCCCCGGAGCGCCGGCGGCTGTCGAGGCGCGGCGAGCCGCA
 
GCCATTGCCTTTTATGGTAATCGTGCGAGAGGGCGCAGGGACTTCCTTTGTCCCAAA
TCTGTGCGGAGCCGAAATCTGGGAGGCGCCGCCGCACCCCCTCTAGCGGGCGCGGGG
CGAAGCGGTGCGGCGCCGGCAGGAAGGAAATGGGCGGGGAGGGCCTTCGTGCGTCGC
CGCGCCGCCGTCCCCTTCTCCCTCTCCAGCCTCGGGGCTGTCCGCGGGGGGACGGCT
GCCTTCGGGGGGGACGGGGCAGGGCGGGGTTCGGCTTCTGGCGTGTGACCGGCGGCT
CTAGAGCCTCTGCTAACCATGTTCATGCCTTCTTCTTTTTCCTACAGCTCCTGGGCA
ACGTGCTGGTTATTGTGCTGTCTCATCATTTTGGCAAAGAATTGCAAGTTTGTACAA
AAAAGCAGGCTGCCACCATGGCCCTGTGGATGCGCTTCCTGCCCCTGCTGGCCCTGC
TCTTCCTCTGGGAGTCCCACCCCACCCAGGCTTTTGTCAAGCAGCACCTTTGTGGTT
CCCACCTGGTGGAGGCTCTCTACCTGGTGTGTGGGGAGCGTGGCTTCTTCTACACAC
CCATGTCCCGCCGTGAAGTGGAGGACCCACAAGTGGCACAACTGGAGCTGGGTGTGA
GCGGCTGGCGGCTGTTCAAGAAGATTAGCGGAGGCCCGGGAGCAGGTGACCTTCAGA
CCTTGGCACTGGAGGTGGCCCAGCAGAAGCGTGGCATTGTAGATCAGTGCTGCACCA
GCATCTGCTCCCTCTACCAGCTGGAGAACTACTGCAACGGAAGCGGAGCCACGAACT
TCTCTCTGTTAAAGCAAGCAGGAGATGTTGAAGAAAACCCCGGGCCTATGGTGAGCA
AGGGCGAGGAGGATAACATGGCCATCATCAAGGAGTTCATGCGCTTCAAGGTGCACA
TGGAGGGCTCCGTGAACGGCCACGAGTTCGAGATCGAGGGCGAGGGCGAGGGCCGCC
CCTACGAGGGCACCCAGACCGCCAAGCTGAAGGTGACCAAGGGTGGCCCCCTGCCCT
TCGCCTGGGACATCCTGTCCCCTCAGTTCATGTACGGCTCCAAGGCCTACGTGAAGC
ACCCCGCCGACATCCCCGACTACTTGAAGCTGTCCTTCCCCGAGGGCTTCAAGTGGG
AGCGCGTGATGAACTTCGAGGACGGCGGCGTGGTGACCGTGACCCAGGACTCCTCCC
TGCAGGACGGCGAGTTCATCTACAAGGTGAAGCTGCGCGGCACCAACTTCCCCTCCG
ACGGCCCCGTAATGCAGAAGAAGACCATGGGCTGGGAGGCCTCCTCCGAGCGGATGT
ACCCCGAGGACGGCGCCCTGAAGGGCGAGATCAAGCAGAGGCTGAAGCTGAAGGACG
GCGGCCACTACGACGCTGAGGTCAAGACCACCTACAAGGCCAAGAAGCCCGTGCAGC
TGCCCGGCGCCTACAACGTCAACATCAAGTTGGACATCACCTCCCACAACGAGGACT
ACACCATCGTGGAACAGTACGAACGCGCCGAGGGCCGCCACTCCACCGGCGGCATGG
 
ACGAGCTGTACAAGTAAACCCAGCTTTCTTGTACAAAGTGGTGATGGCCGGCCGCTT
CGAGCAGACATGATAAGATACATTGATGAGTTTGGACAAACCACAACTAGAATGCAG
TGAAAAAAATGCTTTATTTGTGAAATTTGTGATGCTATTGCTTTATTTGTAACCATT
ATAAGCTGCAATAAACAAGTTAACAACAACAATTGCATTCATTTTATGTTTCAGGTT
CAGGGGGAGGTGTGGGAGGTTTTTTAAAGCAAGTAAAACCTCTACAAATGTGGTAGC
GGCCGCGGCGCTCTTCCGCTTCCTCGCTCACTGACTCGCTGCGCTCGGTCGTTCGGC
TGCGGCGAGCGGTATCAGCTCACTCAAAGGCGGTAATACGGTTATCCACAGAATCAG
GGGATAACGCAGGAAAGAACATGTGAGCAAAAGGCCAGCAAAAGGCCAGGAACCGTA
AAAAGGCCGCGTTGCTGGCGTTTTTCCATAGGCTCCGCCCCCCTGACGAGCATCACA
AAAATCGACGCTCAAGTCAGAGGTGGCGAAACCCGACAGGACTATAAAGATACCAGG
CGTTTCCCCCTGGAAGCTCCCTCGTGCGCTCTCCTGTTCCGACCCTGCCGCTTACCG
GATACCTGTCCGCCTTTCTCTCTTCGGGAAGCGTGGCGCTTTCTCATAGCTCACGCT
GTAGGTATCTCAGTTCGGTGTAGGTCGTTCGCTCCAAGCTGGGCTGTGTGCACGAAC
CCCCCGTTCAGCCCGACCGCTGCGCCTTATCCGGTAACTATCGTCTTGAGTCCAACC
CGGTAAGACACGACTTATCGCCACTGGCAGCAGCCACTGGTAACAGGATTAGCAGAG
CGAGGTATGTAGGCGGTGCTACAGAGTTCTTGAAGTGGTGGCCTAACTACGGCTACA
CTAGAAGAACAGTATTTGGTATCTGCGCTCTGCTGAAGCCAGTTACCTTCGGAAAAA
GAGTTGGTAGCTCTTGATCCGGCAAACAAACCACCGCTGGTAGCGGTGGTTTTTTTG
TTTGCAAGCAGCAGATTACGCGCAGAAAAAAAGGATCTCAAGAAGATCCTTTGATCT
TTTCTACGGGGTCTGACGCTCAGTGGAACGAAAACTCACGTTAAGGGATTTTGGTCA
TGAGATTATCAAAAAGGATCTTCACCTAGATCCTTTTAAATTAAAAATGAAGTTTTA
AATCAATCTAAAGTATATATGAGTAAACTTGGTCTGACAGTTACCAATGCTTAATCA
GTGAGGCACCTATCTCAGCGATCTGTCTATTTCGTTCATCCATAGTTGCCTGACTCC
CCGTCGTGTAGATAACTACGATACGGGAGGGCTTACCATCTGGCCCCAGTGCTGCAA
TGATACCGCGAGACCCACGCTCACCGGCTCCAGATTTATCAGCAATAAACCAGCCAG
CCGGAAGGGCCGAGCGCAGAAGTGGTCCTGCAACTTTATCCGCCTCCATCCAGTCTA
TTAATTGTTGCCGGGAAGCTAGAGTAAGTAGTTCGCCAGTTAATAGTTTGCGCAACG
 
TTGTTGCCATTGCTACAGGCATCGTGGTGTCACGCTCGTCGTTTGGTATGGCTTCAT
TCAGCTCCGGTTCCCAACGATCAAGGCGAGTTACATGATCCCCCATGTTGTGCAAAA
AAGCGGTTAGCTCCTTCGGTCCTCCGATCGTTGTCAGAAGTAAGTTGGCCGCAGTGT
TATCACTCATGGTTATGGCAGCACTGCATAATTCTCTTACTGTCATGCCATCCGTAA
GATGCTTTTCTGTGACTGGTGAGTACTCAACCAAGTCATTCTGAGAATAGTGTATGC
GGCGACCGAGTTGCTCTTGCCCGGCGTCAATACGGGATAATACCGCGCCACATAGCA
GAACTTTAAAAGTGCTCATCATTGGAAAACGTTCTTCGGGGCGAAAACTCTCAAGGA
TCTTACCGCTGTTGAGATCCAGTTCGATGTAACCCACTCGTGCACCCAACTGATCTT
CAGCATCTTTTACTTTCACCAGCGTTTCTGGGTGAGCAAAAACAGGAAGGCAAAATG
CCGCAAAAAAGGGAATAAGGGCGACACGGAAATGTTGAATACTCATACTCTTCCTTT
TTCAATATTATTGAAGCATTTATCAGGGTTATTGTCTCATGAGCGGATACATATTTG
AATGTATTTAGAAAAATAAACAAATAGGGGTTCCGCGCACATTTCCCCGAAAAGTGC
CACCTGACGTCTAAGAAACCATTATTATCATGACATTAACCTATAAAAATAGGCGTA
TCACGAGGCCCTTTCGTCGGCGCGCCGCGGCCGC
SEQ ID NO: 20
   mIns2-HiBiT1-encoding gene 
ATGGCCCTGTGGATGCGCTTCCTGCCCCTGCTGGCCCTGCTCTTCCTCTGGGAGTCC

CACCCCACCCAGGCTTTTGTCAAGCAGCACCTTTGTGGTTCCCACCTGGTGGAGGCT

CTCTACCTGGTGTGTGGGGAGCGTGGCTTCTTCTACACACCCATGTCCCGCCGTGAA

GTGGAGGACCCACAAGTGGCACAACTGGAGCTGGGTGTGAGCGGCTGGCGGCTGTTC

AAGAAGATTAGCGGAGGCCCGGGAGCAGGTGACCTTCAGACCTTGGCACTGGAGGTG

GCCCAGCAGAAGCGTGGCATTGTAGATCAGTGCTGCACCAGCATCTGCTCCCTCTAC

CAGCTGGAGAACTACTGCAAC
SEQ ID NO: 21
   mIns2-HiBiT1 amino acid sequence 
MALWMRFLPLLALLFLWESHPTQAFVKQHLCGSHLVEALYLVCGERGFFYTPMSRRE
VEDPQVAQLELGVSGWRLFKKISGGPGAGDLQTLALEVAQQKRGIVDQCCTSICSLY
QLENYCN
SEQ ID NO: 22
   mIns2-HiBiT2-encoding gene 
ATGGCCCTGTGGATGCGCTTCCTGCCCCTGCTGGCCCTGCTCTTCCTCTGGGAGTCC
CACCCCACCCAGGCTTTTGTCAAGCAGCACCTTTGTGGTTCCCACCTGGTGGAGGCT
CTCTACCTGGTGTGTGGGGAGCGTGGCTTCTTCTACACACCCATGTCCCGCCGTGAA
GTGGAGGACCCACAAGTGGCACAACTGGAGCTGGGTGGAGGCGTGAGCGGCTGGCGG
CTGTTCAAGAAGATTAGCTTGGCACTGGAGGTGGCCCAGCAGAAGCGTGGCATTGTA
GATCAGTGCTGCACCAGCATCTGCTCCCTCTACCAGCTGGAGAACTACTGCAAC
SEQ ID NO: 23
   mIns2-HiBiT2 amino acid sequence 
MALWMRFLPLLALLFLWESHPTQAFVKQHLCGSHLVEALYLVCGERGFFYTPMSRRE
VEDPQVAQLELGGGVSGWRLFKKISLALEVAQQKRGIVDQCCTSICSLYQLENYCN
SEQ ID NO: 24
   mIns2-HiBiT3-encoding gene 
ATGGCCCTGTGGATGCGCTTCCTGCCCCTGCTGGCCCTGCTCTTCCTCTGGGAGTCC

CACCCCACCCAGGCTTTTGTCAAGCAGCACCTTTGTGGTTCCCACCTGGTGGAGGCT

CTCTACCTGGTGTGTGGGGAGCGTGGCTTCTTCTACACACCCATGTCCCGCCGTGAA

GTGGAGGACCCAGTGAGCGGCTGGCGGCTGTTCAAGAAGATTAGCGGTGGAGGCCCG

GGAGCAGGTGACCTTCAGACCTTGGCACTGGAGGTGGCCCAGCAGAAGCGTGGCATT

GTAGATCAGTGCTGCACCAGCATCTGCTCCCTCTACCAGCTGGAGAACTACTGCAAC
SEQ ID NO: 25
   mIns2-HiBiT3 amino acid sequence 
MALWMRFLPLLALLFLWESHPTQAFVKQHLCGSHLVEALYLVCGERGFFYTPMSRRE
VEDPVSGWRLFKKISGGGPGAGDLQTLALEVAQQKRGIVDQCCTSICSLYQLENYCN
SEQ ID NO: 26
   mIns2-HiBiT4-encoding gene 
ATGGCCCTGTGGATGCGCTTCCTGCCCCTGCTGGCCCTGCTCTTCCTCTGGGAGTCC

CACCCCACCCAGGCTTTTGTCAAGCAGCACCTTTGTGGTTCCCACCTGGTGGAGGCT

CTCTACCTGGTGTGTGGGGAGCGTGGCTTCTTCTACACACCCATGTCCCGCCGTGAA

GTGGAGGACCCACAAGTGGCACAACTGGAGCTGGGTGGAGGCGTGAGCGGCTGGCGG

CTGTTCAAGAAGATTAGCCTTCAGACCTTGGCACTGGAGGTGGCCCAGCAGAAGCGT

GGCATTGTAGATCAGTGCTGCACCAGCATCTGCTCCCTCTACCAGCTGGAGAACTAC

TGCAAC
SEQ ID NO: 27
   mIns2-HiBiT4 amino acid sequence 
MALWMRFLPLLALLFLWESHPTQAFVKQHLCGSHLVEALYLVCGERGFFYTPMSRRE
VEDPQVAQLELGGGVSGWRLFKKISLQTLALEVAQQKRGIVDQCCTSICSLYQLENY
CN
SEQ ID NO: 28
   mIns2-HiBiT5-encoding gene 
ATGGCCCTGTGGATGCGCTTCCTGCCCCTGCTGGCCCTGCTCTTCCTCTGGGAGTCC

CACCCCACCCAGGCTTTTGTCAAGCAGCACCTTTGTGGTTCCCACCTGGTGGAGGCT

CTCTACCTGGTGTGTGGGGAGCGTGGCTTCTTCTACACACCCATGTCCCGCCGTGAA

GTGGAGGACCCACAAGTGGTGAGCGGCTGGCGGCTGTTCAAGAAGATTAGCGGTGGA
 
GGCCCGGGAGCAGGTGACCTTCAGACCTTGGCACTGGAGGTGGCCCAGCAGAAGCGT
GGCATTGTAGATCAGTGCTGCACCAGCATCTGCTCCCTCTACCAGCTGGAGAACTAC
TGCAAC
SEQ ID NO: 29
   mIns2-HiBiT5 amino acid sequence 
MALWMRFLPLLALLFLWESHPTQAFVKQHLCGSHLVEALYLVCGERGFFYTPMSRRE
VEDPQVVSGWRLFKKISGGGPGAGDLQTLALEVAQQKRGIVDQCCTSICSLYQLENY
CN
SEQ ID NO: 30
   hIns-HiBiTT1-encoding gene 
ATGGCCCTGTGGATGCGCCTCCTGCCCCTGCTGGCGCTGCTGGCCCTCTGGGGACCT
GACCCAGCCGCAGCCTTTGTGAACCAACACCTGTGCGGCTCACACCTGGTGGAAGCT
CTCTACCTAGTGTGCGGGGAACGAGGCTTCTTCTACACACCCAAGACCCGCCGGGAG
GCAGAGGACCTGCAGGTGGGGCAGGTGGAGCTGGGCGTGAGCGGCTGGCGGCTGTTC
AAGAAGATTAGCGGGGGCCCTGGTGCAGGCAGCCTGCAGCCCTTGGCCCTGGAGGGG
TCCCTGCAGAAGCGTGGCATTGTGGAACAATGCTGTACCAGCATCTGCTCCCTCTAC
CAGCTGGAGAACTACTGCAACTAAGAATTCA
SEQ ID NO: 31
   hIns-HiBiT1 amino acid sequence 
MALWMRLLPLLALLALWGPDPAAAFVNQHLCGSHLVEALYLVCGERGFFYTPKTRRE
AEDLQVGQVELGVSGWRLFKKISGGPGAGSLQPLALEGSLQKRGIVEQCCTSICSLY
QLENYCN
SEQ ID NO: 32
   hIns-HiBiT2-encoding gene 
ATGGCCCTGTGGATGCGCCTCCTGCCCCTGCTGGCGCTGCTGGCCCTCTGGGGACCT

GACCCAGCCGCAGCCTTTGTGAACCAACACCTGTGCGGCTCACACCTGGTGGAAGCT

CTCTACCTAGTGTGCGGGGAACGAGGCTTCTTCTACACACCCAAGACCCGCCGGGAG

GCAGAGGACCTGCAGGTGGGGCAGGTGGAGCTGGGCGGGGGCGTGAGCGGCTGGCGG

CTGTTCAAGAAGATTAGCTTGGCCCTGGAGGGGTCCCTGCAGAAGCGTGGCATTGTG

GAACAATGCTGTACCAGCATCTGCTCCCTCTACCAGCTGGAGAACTACTGCAACTAA

GAATTCA
SEQ ID NO: 33 hIns-HiBiT2-amino acid sequence 
MALWMRLLPLLALLALWGPDPAAAFVNQHLCGSHLVEALYLVCGERGFFYTPKTRRE

AEDLQVGQVELGGGVSGWRLFKKISLALEGSLQKRGIVEQCCTSICSLYQLENYCN
SEQ ID NO: 34
   hIns-HiBiT5-encoding gene 
ATGGCCCTGTGGATGCGCCTCCTGCCCCTGCTGGCGCTGCTGGCCCTCTGGGGACCT
GACCCAGCCGCAGCCTTTGTGAACCAACACCTGTGCGGCTCACACCTGGTGGAAGCT
CTCTACCTAGTGTGCGGGGAACGAGGCTTCTTCTACACACCCAAGACCCGCCGGGAG
GCAGAGGACCTGCAGGTGGTGAGCGGCTGGCGGCTGTTCAAGAAGATTAGCGGCGG
GGGCCCTGGTGCAGGCAGCCTGCAGCCCTTGGCCCTGGAGGGGTCCCTGCAGAAGCGT
GGCATTGTGGAACAATGCTGTACCAGCATCTGCTCCCTCTACCAGCTGGAGAACTAC
TGCAACTAAGAATTCA
SEQ ID NO: 35
   hIns-HiBiT5 amino acid sequence 
MALWMRLLPLLALLALWGPDPAAAFVNQHLCGSHLVEALYLVCGERGFFYTPKTRRE

AEDLQVVSGWRLFKKISGGGPGAGSLQPLALEGSLQKRGIVEQCCTSICSLYQLENY

CN
SEQ ID NO: 36
   Pre-Donor-arm1-HiBiT-arm2 plasmid 
TCTAGATAGCATCGTACGCGTACGTGTTTGGCCCAGATCACTGTCCTTCTGCCGGAT
CCATGGTGAGCGGCTGGCGGCTGTTCAAGAAGATTAGCTAAGAATTCAAGGCCTCTC
GAGCCACTATAGTGAGTCGTATTACGTAGATCCAGACATGATAAGATACATTGATGA
GTTTGGACAAACCACAACTAGAATGCAGTGAAAAAAATGCTTTATTTGTGAAATTTG
TGATGCTATTGCTTTATTTGTAACCATTATAAGCTGCAATAAACAAGTTAACAACAA
CAATTGCATTCATTTTATGTTTCAGGTTCAGGGGGAGGTGTGGGAGGTTTTTTAACG
CGCCGCACACAAAAACCAACACACAGATCATGAAAATAAAGCTCTTTTATTGGTACC
GAATTCGCCAGGGAGCTCTCAGACGTCGCTTGGTCGGTCTTTATTCGAACCCCAGAG
TCCCGCTCACATGGTTTAGTTCCTCACCTTGTCGTATTATACTATGCCGATATACTA
TGCCGATGATTAATTGTCAACAGGCTGCAGGTCGAAAGGCCCGGAGATGAGGAAGAG
GAGAACAGCGCGGCAGACGTGCGCTTTTGAAGCGTGCAGAATGCCGGGCCTCCGGAG
GACCTTCGGGCGCCCGCCCCGCCCCTGAGCCCGCCCCTGAGCCCGCCCCCGGACCCA
CCCCTTCCCAGCCTCTGAGCCCAGAAAGCGAAGGAGCAAAGCTGCTATTGGCCGCTG
CCCCAAAGGCCTACCCGCTTCCATTGCTCAGCGGTGCTGTCCATCTGCACGAGACTA
GTGAGACGTGCTACTTCCATTTGTCACGTCCTGCACGACGCGAGCTGCGGGGCGGG
GGGGAACTTCCTGACTAGGGGAGGAGTAGAAGGTGGCGCGAAGGGGCCACCAAAGAAC
GGAGCCGGTTGGCGCCTACCGGTGGATGTGGAATGTGTGCGAGGCCAGAGGCCACTT
GTGTAGCGCCAAGTGCCCAGCGGGGCTGCTAAAGCGCATGCTCCAGACTGCCTTGGG
AAAAGCGCCTCCCCTACCCGGTAGAATCCATGGCCCTGTGGATGCGCCCAAACACGT
ACGCGTACGATGCTCAAGCTTGGCGCTCTTCCGCTTCCTCGCTCACTGACTCGCTGC
GCTCGGTCGTTCGGCTGCGGCGAGCGGTATCAGCTCACTCAAAGGCGGTAATACGGT
 
TATCCACAGAATCAGGGGATAACGCAGGAAAGAACATGTGAGCAAAAGGCCAGCAAA
AGGCCAGGAACCGTAAAAAGGCCGCGTTGCTGGCGTTTTTCCATAGGCTCCGCCCCC
CTGACGAGCATCACAAAAATCGACGCTCAAGTCAGAGGTGGCGAAACCCGACAGGAC
TATAAAGATACCAGGCGTTTCCCCCTGGAAGCTCCCTCGTGCGCTCTCCTGTTCCGA
CCCTGCCGCTTACCGGATACCTGTCCGCCTTTCTCCCTTCGGGAAGCGTGGCGCTTT
CTCATAGCTCACGCTGTAGGTATCTCAGTTCGGTGTAGGTCGTTCGCTCCAAGCTGG
GCTGTGTGCACGAACCCCCCGTTCAGCCCGACCGCTGCGCCTTATCCGGTAACTATC
GTCTTGAGTCCAACCCGGTAAGACACGACTTATCGCCACTGGCAGCAGCCACTGGTA
ACAGGATTAGCAGAGCGAGGTATGTAGGCGGTGCTACAGAGTTCTTGAAGTGGTGGC
CTAACTACGGCTACACTAGAAGAACAGTATTTGGTATCTGCGCTCTGCTGAAGCCAG
TTACCTTCGGAAAAAGAGTTGGTAGCTCTTGATCCGGCAAACAAACCACCGCTGGTA
GCGGTGGTTTTTTTGTTTGCAAGCAGCAGATTACGCGCAGAAAAAAAGGATCTCAAG
AAGATCCTTTGATCTTTTCTACGGGGTCTGACGCTCAGTGGAACGAAAACTCACGTT
AAGGGATTTTGGTCATGAGATTATCAAAAAGGATCTTCACCTAGATCCTTTTAAATT
AAAAATGAAGTTTTAAATCAATCTAAAGTATATATGAGTAAACTTGGTCTGACAGTT
ACCAATGCTTAATCAGTGAGGCACCTATCTCAGCGATCTGTCTATTTCGTTCATCCA
TAGTTGCCTGACTCCCCGTCGTGTAGATAACTACGATACGGGAGGGCTTACCATCTG
GCCCCAGTGCTGCAATGATACCGCGAGATCCACGCTCACCGGCTCCAGATTTATCAG
CAATAAACCAGCCAGCCGGAAGGGCCGAGCGCAGAAGTGGTCCTGCAACTTTATCCG
CCTCCATCCAGTCTATTAATTGTTGCCGGGAAGCTAGAGTAAGTAGTTCGCCAGTTA
ATAGTTTGCGCAACGTTGTTGCCATTGCTACAGGCATCGTGGTGTCACGCTCGTCGT
TTGGTATGGCTTCATTCAGCTCCGGTTCCCAACGATCAAGGCGAGTTACATGATCCC
CCATGTTGTGCAAAAAAGCGGTTAGCTCCTTCGGTCCTCCGATCGTTGTCAGAAGTA
AGTTGGCCGCAGTGTTATCACTCATGGTTATGGCAGCACTGCATAATTCTCTTACTG
TCATGCCATCCGTAAGATGCTTTTCTGTGACTGGTGAGTACTCAACCAAGTCATTCT
GAGAATAGTGTATGCGGCGACCGAGTTGCTCTTGCCCGGCGTCAATACGGGATAATA
CCGCGCCACATAGCAGAACTTTAAAAGTGCTCATCATTGGAAAACGTTCTTCGGGGC
 
GAAAACTCTCAAGGATCTTACCGCTGTTGAGATCCAGTTCGATGTAACCCACTCGTG
CACCCAACTGATCTTCAGCATCTTTTACTTTCACCAGCGTTTCTGGGTGAGCAAAAA
CAGGAAGGCAAAATGCCGCAAAAAAGGGAATAAGGGCGACACGGAAATGTTGAATAC
TCATACTCTTCCTTTTTCAATATTATTGAAGCATTTATCAGGGTTATTGTCTCATGA
GCGGATACATATTTGAATGTATTTAGAAAAATAAACAAATAGGGGTTCCGCGCACAT
TTCCCCGAAAAGTGCCACCTGACGTCTAAGAAACCATTATTATCATGACATTAACCT
ATAAAAATAGGCGTATCACGAGGCCCTTTCGTC
SEQ ID NO: 37
   Primer neoRdonorF
   CCGATCCCATGGTTTAGTTCCTCACCTTG
SEQ ID NO: 38
   Primer neoRdonorR
   CTTCTGAGCGGGACTCTGGGGTTC
SEQ ID NO: 39
   Primer inFneoRr
   AAACCATGGGATCGGCCATTGAACAAG
SEQ ID NO: 40
   Primer inFneoRf
   AGTCCCGCTCAGAAGAACTCGTCAAGAAGG
SEQ ID NO: 41
   Pre-Donor-arm1-NeoR-arm2 
TCTAGATAGCATCGTACGCGTACGTGTTTGGCCCAGATCACTGTCCTTCTGCCGGAT
CCATGGTGAGCGGCTGGCGGCTGTTCAAGAAGATTAGCTAAGAATTCAAGGCCTCTC
GAGCCACTATAGTGAGTCGTATTACGTAGATCCAGACATGATAAGATACATTGATGA
GTTTGGACAAACCACAACTAGAATGCAGTGAAAAAAATGCTTTATTTGTGAAATTTG
TGATGCTATTGCTTTATTTGTAACCATTATAAGCTGCAATAAACAAGTTAACAACAA
CAATTGCATTCATTTTATGTTTCAGGTTCAGGGGGAGGTGTGGGAGGTTTTTTAACG
CGCCGCACACAAAAACCAACACACAGATCATGAAAATAAAGCTCTTTTATTGGTACC
GAATTCGCCAGGGAGCTCTCAGACGTCGCTTGGTCGGTCTTTATTCGAACCCCAGAG
TCCCGCTCAGAAGAACTCGTCAAGAAGGCGATAGAAGGCGATGCGCTGCGAATCGGG
AGCGGCGATACCGTAAAGCACGAGGAAGCGGTCAGCCCATTCGCCGCCAAGCTCTTC
AGCAATATCACGGGTAGCCAACGCTATGTCCTGATAGCGGTCCGCCACACCCAGCCG
GCCACAGTCGATGAATCCAGAAAAGCGGCCATTTTCCACCATGATATTCGGCAAGCA
GGCATCGCCATGGGTCACGACGAGATCCTCGCCGTCGGGCATGCGCGCCTTGAGCCT
GGCGAACAGTTCGGCTGGCGCGAGCCCCTGATGCTCTTCGTCCAGATCATCCTGATC
GACAAGACCGGCTTCCATCCGAGTACGTGCTCGCTCGATGCGATGTTTCGCTTGGTG
GTCGAATGGGCAGGTAGCCGGATCAAGCGTATGCAGCCGCCGCATTGCATCAGCCAT
GATGGATACTTTCTCGGCAGGAGCAAGGTGAGATGACAGGAGATCCTGCCCCGGCAC
TTCGCCCAATAGCAGCCAGTCCCTTCCCGCTTCAGTGACAACGTCGAGCACAGCTGC
GCAAGGAACGCCCGTCGTGGCCAGCCACGATAGCCGCGCTGCCTCGTCCTGCAGTTC
ATTCAGGGCACCGGACAGGTCGGTCTTGACAAAAAGAACCGGGCGCCCCTGCGCTGA
CAGCCGGAACACGGCGGCATCAGAGCAGCCGATTGTCTGTTGTGCCCAGTCATAGCC
GAATAGCCTCTCCACCCAAGCGGCCGGAGAACCTGCGTGCAATCCATCTTGTTCAAT
GGCCGATCCCATGGTTTAGTTCCTCACCTTGTCGTATTATACTATGCCGATATACTA
TGCCGATGATTAATTGTCAACAGGCTGCAGGTCGAAAGGCCCGGAGATGAGGAAGAG
GAGAACAGCGCGGCAGACGTGCGCTTTTGAAGCGTGCAGAATGCCGGGCCTCCGGAG
GACCTTCGGGCGCCCGCCCCGCCCCTGAGCCCGCCCCTGAGCCCGCCCCCGGACCCA
CCCCTTCCCAGCCTCTGAGCCCAGAAAGCGAAGGAGCAAAGCTGCTATTGGCCGCTG
 
CCCCAAAGGCCTACCCGCTTCCATTGCTCAGCGGTGCTGTCCATCTGCACGAGACTA
GTGAGACGTGCTACTTCCATTTGTCACGTCCTGCACGACGCGAGCTGCGGGGCGGGG
GGGAACTTCCTGACTAGGGGAGGAGTAGAAGGTGGCGCGAAGGGGCCACCAAAGAAC
GGAGCCGGTTGGCGCCTACCGGTGGATGTGGAATGTGTGCGAGGCCAGAGGCCACTT
GTGTAGCGCCAAGTGCCCAGCGGGGCTGCTAAAGCGCATGCTCCAGACTGCCTTGGG
AAAAGCGCCTCCCCTACCCGGTAGAATCCATGGCCCTGTGGATGCGCCCAAACACGT
ACGCGTACGATGCTCAAGCTT
SEQ ID NO: 42
   Primer DonorR
   CCATGGATCCGGCAGAAGGAC
SEQ ID NO: 43
   Primer DonorF
   CTAAGAATTCAAGGCCTCTCG
SEQ ID NO: 44
   Primer hInsHiBiTF
   CTGCCGGATCCATGGCCCTGTG
SEQ ID NO: 45
   Primer hInsHiBiTR
   CGAGAGGCCTTGAATTCTTAG
SEQ ID NO: 46
   Arm1-hIns-HiBiT1-NeoR-Arm2 
TCTAGATAGCATCGTACGCGTACGTGTTTGGCCCAGATCACTGTCCTTCTGCCGGAT
CCATGGCCCTGTGGATGCGCCTCCTGCCCCTGCTGGCGCTGCTGGCCCTCTGGGGAC
CTGACCCAGCCGCAGCCTTTGTGAACCAACACCTGTGCGGCTCACACCTGGTGGAAG
CTCTCTACCTAGTGTGCGGGGAACGAGGCTTCTTCTACACACCCAAGACCCGCCGGG
AGGCAGAGGACCTGCAGGTGGGGCAGGTGGAGCTGGGCGTGAGCGGCTGGCGGCTGT
TCAAGAAGATTAGCGGGGGCCCTGGTGCAGGCAGCCTGCAGCCCTTGGCCCTGGAGG
GGTCCCTGCAGAAGCGTGGCATTGTGGAACAATGCTGTACCAGCATCTGCTCCCTCT
ACCAGCTGGAGAACTACTGCAACTAAGAATTCAAGGCCTCTCGAGCCACTATAGTGA
GTCGTATTACGTAGATCCAGACATGATAAGATACATTGATGAGTTTGGACAAACCAC
AACTAGAATGCAGTGAAAAAAATGCTTTATTTGTGAAATTTGTGATGCTATTGCTTT
ATTTGTAACCATTATAAGCTGCAATAAACAAGTTAACAACAACAATTGCATTCATTT
TATGTTTCAGGTTCAGGGGGAGGTGTGGGAGGTTTTTTAACGCGCCGCACACAAAAA
CCAACACACAGATCATGAAAATAAAGCTCTTTTATTGGTACCGAATTCGCCAGGGAG
CTCTCAGACGTCGCTTGGTCGGTCTTTATTCGAACCCCAGAGTCCCGCTCAGAAGAA
CTCGTCAAGAAGGCGATAGAAGGCGATGCGCTGCGAATCGGGAGCGGCGATACCGTA
AAGCACGAGGAAGCGGTCAGCCCATTCGCCGCCAAGCTCTTCAGCAATATCACGGGT
AGCCAACGCTATGTCCTGATAGCGGTCCGCCACACCCAGCCGGCCACAGTCGATGAA
TCCAGAAAAGCGGCCATTTTCCACCATGATATTCGGCAAGCAGGCATCGCCATGGGT
CACGACGAGATCCTCGCCGTCGGGCATGCGCGCCTTGAGCCTGGCGAACAGTTCGGC
TGGCGCGAGCCCCTGATGCTCTTCGTCCAGATCATCCTGATCGACAAGACCGGCTTC
CATCCGAGTACGTGCTCGCTCGATGCGATGTTTCGCTTGGTGGTCGAATGGGCAGGT
AGCCGGATCAAGCGTATGCAGCCGCCGCATTGCATCAGCCATGATGGATACTTTCTC
GGCAGGAGCAAGGTGAGATGACAGGAGATCCTGCCCCGGCACTTCGCCCAATAGCAG
CCAGTCCCTTCCCGCTTCAGTGACAACGTCGAGCACAGCTGCGCAAGGAACGCCCGT
CGTGGCCAGCCACGATAGCCGCGCTGCCTCGTCCTGCAGTTCATTCAGGGCACCGGA
CAGGTCGGTCTTGACAAAAAGAACCGGGCGCCCCTGCGCTGACAGCCGGAACACGGC
GGCATCAGAGCAGCCGATTGTCTGTTGTGCCCAGTCATAGCCGAATAGCCTCTCCAC
 
CCAAGCGGCCGGAGAACCTGCGTGCAATCCATCTTGTTCAATGGCCGATCCCATGGT
TTAGTTCCTCACCTTGTCGTATTATACTATGCCGATATACTATGCCGATGATTAATT
GTCAACAGGCTGCAGGTCGAAAGGCCCGGAGATGAGGAAGAGGAGAACAGCGCGGCA
GACGTGCGCTTTTGAAGCGTGCAGAATGCCGGGCCTCCGGAGGACCTTCGGGCGCCC
GCCCCGCCCCTGAGCCCGCCCCTGAGCCCGCCCCCGGACCCACCCCTTCCCAGCCTC
TGAGCCCAGAAAGCGAAGGAGCAAAGCTGCTATTGGCCGCTGCCCCAAAGGCCTACC
CGCTTCCATTGCTCAGCGGTGCTGTCCATCTGCACGAGACTAGTGAGACGTGCTACT
TCCATTTGTCACGTCCTGCACGACGCGAGCTGCGGGGCGGGGGGGAACTTCCTGACT
AGGGGAGGAGTAGAAGGTGGCGCGAAGGGGCCACCAAAGAACGGAGCCGGTTGGCGC
CTACCGGTGGATGTGGAATGTGTGCGAGGCCAGAGGCCACTTGTGTAGCGCCAAGTG
CCCAGCGGGGCTGCTAAAGCGCATGCTCCAGACTGCCTTGGGAAAAGCGCCTCCCCT
ACCCGGTAGAATCCATGGCCCTGTGGATGCGCCCAAACACGTACGCGTACGATGCTC
AAGCTT
SEQ ID NO: 47
   Guide RNA-hCAS9 plasmid 
GAGGGCCTATTTCCCATGATTCCTTCATATTTGCATATACGATACAAGGCTGTTAGA

GAGATAATTGGAATTAATTTGACTGTAAACACAAAGATATTAGTACAAAATACGTGA

CGTAGAAAGTAATAATTTCTTGGGTAGTTTGCAGTTTTAAAATTATGTTTTAAAATG

GACTATCATATGCTTACCGTAACTTGAAAGTATTTCGATTTCTTGGCTTTATATATC

TTGTGGAAAGGACGAAACACCGAGATCACTGTCCTTCTGCCAGTTTTAGAGCTAGAA

ATAGCAAGTTAAAATAAGGCTAGTCCGTTATCAACTTGAAAAAGTGGCACCGAGTCG

GTGCTTTTTTGGCCGGCCGAGGGCCTATTTCCCATGATTCCTTCATATTTGCATATA

CGATACAAGGCTGTTAGAGAGATAATTGGAATTAATTTGACTGTAAACACAAAGATA

TTAGTACAAAATACGTGACGTAGAAAGTAATAATTTCTTGGGTAGTTTGCAGTTTTA

AAATTATGTTTTAAAATGGACTATCATATGCTTACCGTAACTTGAAAGTATTTCGAT

TTCTTGGCTTTATATATCTTGTGGAAAGGACGAAACACCGGCATCGTACGCGTACGT
 
GTTGTTTTAGAGCTAGAAATAGCAAGTTAAAATAAGGCTAGTCCGTTATCAACTTGA
AAAAGTGGCACCGAGTCGGTGCTTTTTTGTCTAGAGGTACCCGTTACATAACTTACG
GTAAATGGCCCGCCTGGCTGACCGCCCAACGACCCCCGCCCATTGACGTCAATAGTA
ACGCCAATAGGGACTTTCCATTGACGTCAATGGGTGGAGTATTTACGGTAAACTGCC
CACTTGGCAGTACATCAAGTGTATCATATGCCAAGTACGCCCCCTATTGACGTCAAT
GACGGTAAATGGCCCGCCTGGCATTGTGCCCAGTACATGACCTTATGGGACTTTCCT
ACTTGGCAGTACATCTACGTATTAGTCATCGCTATTACCATGGTCGAGGTGAGCCCC
ACGTTCTGCTTCACTCTCCCCATCTCCCCCCCCTCCCCACCCCCAATTTTGTATTTA
TTTATTTTTTAATTATTTTGTGCAGCGATGGGGGCGGGGGGGGGGGGGGGGCGCGCG
CCAGGCGGGGCGGGGCGGGGCGAGGGGCGGGGCGGGGCGAGGCGGAGAGGTGCGGCG
GCAGCCAATCAGAGCGGCGCGCTCCGAAAGTTTCCTTTTATGGCGAGGCGGCGGCGG
CGGCGGCCCTATAAAAAGCGAAGCGCGCGGCGGGCGGGAGTCGCTGCGCGCTGCCTT
CGCCCCGTGCCCCGCTCCGCCGCCGCCTCGCGCCGCCCGCCCCGGCTCTGACTGACC
GCGTTACTCCCACAGGTGAGCGGGCGGGACGGCCCTTCTCCTCCGGGCTGTAATTAG
CTGAGCAAGAGGTAAGGGTTTAAGGGATGGTTGGTTGGTGGGGTATTAATGTTTAAT
TACCTGGAGCACCTGCCTGAAATCACTTTTTTTCAGGTTGGACCGGTGCCACCATGG
ACTATAAGGACCACGACGGAGACTACAAGGATCATGATATTGATTACAAAGACGATG
ACGATAAGATGGCCCCAAAGAAGAAGCGGAAGGTCGGTATCCACGGAGTCCCAGCAG
CCGACAAGAAGTACAGCATCGGCCTGGACATCGGCACCAACTCTGTGGGCTGGGCCG
TGATCACCGACGAGTACAAGGTGCCCAGCAAGAAATTCAAGGTGCTGGGCAACACCG
ACCGGCACAGCATCAAGAAGAACCTGATCGGAGCCCTGCTGTTCGACAGCGGCGAAA
CAGCCGAGGCCACCCGGCTGAAGAGAACCGCCAGAAGAAGATACACCAGACGGAAGA
ACCGGATCTGCTATCTGCAAGAGATCTTCAGCAACGAGATGGCCAAGGTGGACGACA
GCTTCTTCCACAGACTGGAAGAGTCCTTCCTGGTGGAAGAGGATAAGAAGCACGAGC
GGCACCCCATCTTCGGCAACATCGTGGACGAGGTGGCCTACCACGAGAAGTACCCCA
CCATCTACCACCTGAGAAAGAAACTGGTGGACAGCACCGACAAGGCCGACCTGCGGC
TGATCTATCTGGCCCTGGCCCACATGATCAAGTTCCGGGGCCACTTCCTGATCGAGG
 
GCGACCTGAACCCCGACAACAGCGACGTGGACAAGCTGTTCATCCAGCTGGTGCAGA
CCTACAACCAGCTGTTCGAGGAAAACCCCATCAACGCCAGCGGCGTGGACGCCAAGG
CCATCCTGTCTGCCAGACTGAGCAAGAGCAGACGGCTGGAAAATCTGATCGCCCAGC
TGCCCGGCGAGAAGAAGAATGGCCTGTTCGGAAACCTGATTGCCCTGAGCCTGGGCC
TGACCCCCAACTTCAAGAGCAACTTCGACCTGGCCGAGGATGCCAAACTGCAGCTGA
GCAAGGACACCTACGACGACGACCTGGACAACCTGCTGGCCCAGATCGGCGACCAGT
ACGCCGACCTGTTTCTGGCCGCCAAGAACCTGTCCGACGCCATCCTGCTGAGCGACA
TCCTGAGAGTGAACACCGAGATCACCAAGGCCCCCCTGAGCGCCTCTATGATCAAGA
GATACGACGAGCACCACCAGGACCTGACCCTGCTGAAAGCTCTCGTGCGGCAGCAGC
TGCCTGAGAAGTACAAAGAGATTTTCTTCGACCAGAGCAAGAACGGCTACGCCGGCT
ACATTGACGGCGGAGCCAGCCAGGAAGAGTTCTACAAGTTCATCAAGCCCATCCTGG
AAAAGATGGACGGCACCGAGGAACTGCTCGTGAAGCTGAACAGAGAGGACCTGCTGC
GGAAGCAGCGGACCTTCGACAACGGCAGCATCCCCCACCAGATCCACCTGGGAGAGC
TGCACGCCATTCTGCGGCGGCAGGAAGATTTTTACCCATTCCTGAAGGACAACCGGG
AAAAGATCGAGAAGATCCTGACCTTCCGCATCCCCTACTACGTGGGCCCTCTGGCCA
GGGGAAACAGCAGATTCGCCTGGATGACCAGAAAGAGCGAGGAAACCATCACCCCCT
GGAACTTCGAGGAAGTGGTGGACAAGGGCGCTTCCGCCCAGAGCTTCATCGAGCGGA
TGACCAACTTCGATAAGAACCTGCCCAACGAGAAGGTGCTGCCCAAGCACAGCCTGC
TGTACGAGTACTTCACCGTGTATAACGAGCTGACCAAAGTGAAATACGTGACCGAGG
GAATGAGAAAGCCCGCCTTCCTGAGCGGCGAGCAGAAAAAGGCCATCGTGGACCTGC
TGTTCAAGACCAACCGGAAAGTGACCGTGAAGCAGCTGAAAGAGGACTACTTCAAGA
AAATCGAGTGCTTCGACTCCGTGGAAATCTCCGGCGTGGAAGATCGGTTCAACGCCT
CCCTGGGCACATACCACGATCTGCTGAAAATTATCAAGGACAAGGACTTCCTGGACA
ATGAGGAAAACGAGGACATTCTGGAAGATATCGTGCTGACCCTGACACTGTTTGAGG
ACAGAGAGATGATCGAGGAACGGCTGAAAACCTATGCCCACCTGTTCGACGACAAAG
TGATGAAGCAGCTGAAGCGGCGGAGATACACCGGCTGGGGCAGGCTGAGCCGGAAGC
TGATCAACGGCATCCGGGACAAGCAGTCCGGCAAGACAATCCTGGATTTCCTGAAGT
 
CCGACGGCTTCGCCAACAGAAACTTCATGCAGCTGATCCACGACGACAGCCTGACCT
TTAAAGAGGACATCCAGAAAGCCCAGGTGTCCGGCCAGGGCGATAGCCTGCACGAGC
ACATTGCCAATCTGGCCGGCAGCCCCGCCATTAAGAAGGGCATCCTGCAGACAGTGA
AGGTGGTGGACGAGCTCGTGAAAGTGATGGGCCGGCACAAGCCCGAGAACATCGTGA
TCGAAATGGCCAGAGAGAACCAGACCACCCAGAAGGGACAGAAGAACAGCCGCGAGA
GAATGAAGCGGATCGAAGAGGGCATCAAAGAGCTGGGCAGCCAGATCCTGAAAGAAC
ACCCCGTGGAAAACACCCAGCTGCAGAACGAGAAGCTGTACCTGTACTACCTGCAGA
ATGGGCGGGATATGTACGTGGACCAGGAACTGGACATCAACCGGCTGTCCGACTACG
ATGTGGACCATATCGTGCCTCAGAGCTTTCTGAAGGACGACTCCATCGACAACAAGG
TGCTGACCAGAAGCGACAAGAACCGGGGCAAGAGCGACAACGTGCCCTCCGAAGAGG
TCGTGAAGAAGATGAAGAACTACTGGCGGCAGCTGCTGAACGCCAAGCTGATTACCC
AGAGAAAGTTCGACAATCTGACCAAGGCCGAGAGAGGCGGCCTGAGCGAACTGGATA
AGGCCGGCTTCATCAAGAGACAGCTGGTGGAAACCCGGCAGATCACAAAGCACGTGG
CACAGATCCTGGACTCCCGGATGAACACTAAGTACGACGAGAATGACAAGCTGATCC
GGGAAGTGAAAGTGATCACCCTGAAGTCCAAGCTGGTGTCCGATTTCCGGAAGGATT
TCCAGTTTTACAAAGTGCGCGAGATCAACAACTACCACCACGCCCACGACGCCTACC
TGAACGCCGTCGTGGGAACCGCCCTGATCAAAAAGTACCCTAAGCTGGAAAGCGAGT
TCGTGTACGGCGACTACAAGGTGTACGACGTGCGGAAGATGATCGCCAAGAGCGAGC
AGGAAATCGGCAAGGCTACCGCCAAGTACTTCTTCTACAGCAACATCATGAACTTTT
TCAAGACCGAGATTACCCTGGCCAACGGCGAGATCCGGAAGCGGCCTCTGATCGAGA
CAAACGGCGAAACCGGGGAGATCGTGTGGGATAAGGGCCGGGATTTTGCCACCGTGC
GGAAAGTGCTGAGCATGCCCCAAGTGAATATCGTGAAAAAGACCGAGGTGCAGACAG
GCGGCTTCAGCAAAGAGTCTATCCTGCCCAAGAGGAACAGCGATAAGCTGATCGCCA
GAAAGAAGGACTGGGACCCTAAGAAGTACGGCGGCTTCGACAGCCCCACCGTGGCCT
ATTCTGTGCTGGTGGTGGCCAAAGTGGAAAAGGGCAAGTCCAAGAAACTGAAGAGTG
TGAAAGAGCTGCTGGGGATCACCATCATGGAAAGAAGCAGCTTCGAGAAGAATCCCA
TCGACTTTCTGGAAGCCAAGGGCTACAAAGAAGTGAAAAAGGACCTGATCATCAAGC
 
TGCCTAAGTACTCCCTGTTCGAGCTGGAAAACGGCCGGAAGAGAATGCTGGCCTCTG
CCGGCGAACTGCAGAAGGGAAACGAACTGGCCCTGCCCTCCAAATATGTGAACTTCC
TGTACCTGGCCAGCCACTATGAGAAGCTGAAGGGCTCCCCCGAGGATAATGAGCAGA
AACAGCTGTTTGTGGAACAGCACAAGCACTACCTGGACGAGATCATCGAGCAGATCA
GCGAGTTCTCCAAGAGAGTGATCCTGGCCGACGCTAATCTGGACAAAGTGCTGTCCG
CCTACAACAAGCACCGGGATAAGCCCATCAGAGAGCAGGCCGAGAATATCATCCACC
TGTTTACCCTGACCAATCTGGGAGCCCCTGCCGCCTTCAAGTACTTTGACACCACCA
TCGACCGGAAGAGGTACACCAGCACCAAAGAGGTGCTGGACGCCACCCTGATCCACC
AGAGCATCACCGGCCTGTACGAGACACGGATCGACCTGTCTCAGCTGGGAGGCGACA
AAAGGCCGGCGGCCACGAAAAAGGCCGGCCAGGCAAAAAAGAAAAAGTAAGAATTCC
TAGAGCTCGCTGATCAGCCTCGACTGTGCCTTCTAGTTGCCAGCCATCTGTTGTTTG
CCCCTCCCCCGTGCCTTCCTTGACCCTGGAAGGTGCCACTCCCACTGTCCTTTCCTA
ATAAAATGAGGAAATTGCATCGCATTGTCTGAGTAGGTGTCATTCTATTCTGGGGGG
TGGGGTGGGGCAGGACAGCAAGGGGGAGGATTGGGAAGAGAATAGCAGGCATGCTGG
GGAGCGGCCGCAGGAACCCCTAGTGATGGAGTTGGCCACTCCCTCTCTGCGCGCTCG
CTCGCTCACTGAGGCCGGGCGACCAAAGGTCGCCCGACGCCCGGGCTTTGCCCGGGC
GGCCTCAGTGAGCGAGCGAGCGCGCAGCTGCCTGCAGGGGCGCCTGATGCGGTATTT
TCTCCTTACGCATCTGTGCGGTATTTCACACCGCATACGTCAAAGCAACCATAGTAC
GCGCCCTGTAGCGGCGCATTAAGCGCGGCGGGTGTGGTGGTTACGCGCAGCGTGACC
GCTACACTTGCCAGCGCCTTAGCGCCCGCTCCTTTCGCTTTCTTCCCTTCCTTTCTC
GCCACGTTCGCCGGCTTTCCCCGTCAAGCTCTAAATCGGGGGCTCCCTTTAGGGTTC
CGATTTAGTGCTTTACGGCACCTCGACCCCAAAAAACTTGATTTGGGTGATGGTTCA
CGTAGTGGGCCATCGCCCTGATAGACGGTTTTTCGCCCTTTGACGTTGGAGTCCACG
TTCTTTAATAGTGGACTCTTGTTCCAAACTGGAACAACACTCAACTCTATCTCGGGC
TATTCTTTTGATTTATAAGGGATTTTGCCGATTTCGGTCTATTGGTTAAAAAATGAG
CTGATTTAACAAAAATTTAACGCGAATTTTAACAAAATATTAACGTTTACAATTTTA
TGGTGCACTCTCAGTACAATCTGCTCTGATGCCGCATAGTTAAGCCAGCCCCGACAC
 
CCGCCAACACCCGCTGACGCGCCCTGACGGGCTTGTCTGCTCCCGGCATCCGCTTAC
AGACAAGCTGTGACCGTCTCCGGGAGCTGCATGTGTCAGAGGTTTTCACCGTCATCA
CCGAAACGCGCGAGACGAAAGGGCCTCGTGATACGCCTATTTTTATAGGTTAATGTC
ATGATAATAATGGTTTCTTAGACGTCAGGTGGCACTTTTCGGGGAAATGTGCGCGGA
ACCCCTATTTGTTTATTTTTCTAAATACATTCAAATATGTATCCGCTCATGAGACAA
TAACCCTGATAAATGCTTCAATAATATTGAAAAAGGAAGAGTATGAGTATTCAACAT
TTCCGTGTCGCCCTTATTCCCTTTTTTGCGGCATTTTGCCTTCCTGTTTTTGCTCAC
CCAGAAACGCTGGTGAAAGTAAAAGATGCTGAAGATCAGTTGGGTGCACGAGTGGGT
TACATCGAACTGGATCTCAACAGCGGTAAGATCCTTGAGAGTTTTCGCCCCGAAGAA
CGTTTTCCAATGATGAGCACTTTTAAAGTTCTGCTATGTGGCGCGGTATTATCCCGT
ATTGACGCCGGGCAAGAGCAACTCGGTCGCCGCATACACTATTCTCAGAATGACTTG
GTTGAGTACTCACCAGTCACAGAAAAGCATCTTACGGATGGCATGACAGTAAGAGAA
TTATGCAGTGCTGCCATAACCATGAGTGATAACACTGCGGCCAACTTACTTCTGACA
ACGATCGGAGGACCGAAGGAGCTAACCGCTTTTTTGCACAACATGGGGGATCATGTA
ACTCGCCTTGATCGTTGGGAACCGGAGCTGAATGAAGCCATACCAAACGACGAGCGT
GACACCACGATGCCTGTAGCAATGGCAACAACGTTGCGCAAACTATTAACTGGCGAA
CTACTTACTCTAGCTTCCCGGCAACAATTAATAGACTGGATGGAGGCGGATAAAGTT
GCAGGACCACTTCTGCGCTCGGCCCTTCCGGCTGGCTGGTTTATTGCTGATAAATCT
GGAGCCGGTGAGCGTGGAAGCCGCGGTATCATTGCAGCACTGGGGCCAGATGGTAAG
CCCTCCCGTATCGTAGTTATCTACACGACGGGGAGTCAGGCAACTATGGATGAACGA
AATAGACAGATCGCTGAGATAGGTGCCTCACTGATTAAGCATTGGTAACTGTCAGAC
CAAGTTTACTCATATATACTTTAGATTGATTTAAAACTTCATTTTTAATTTAAAAGG
ATCTAGGTGAAGATCCTTTTTGATAATCTCATGACCAAAATCCCTTAACGTGAGTTT
TCGTTCCACTGAGCGTCAGACCCCGTAGAAAAGATCAAAGGATCTTCTTGAGATCCT
TTTTTTCTGCGCGTAATCTGCTGCTTGCAAACAAAAAAACCACCGCTACCAGCGGTG
GTTTGTTTGCCGGATCAAGAGCTACCAACTCTTTTTCCGAAGGTAACTGGCTTCAGC
AGAGCGCAGATACCAAATACTGTTCTTCTAGTGTAGCCGTAGTTAGGCCACCACTTC
 
AAGAACTCTGTAGCACCGCCTACATACCTCGCTCTGCTAATCCTGTTACCAGTGGCT
GCTGCCAGTGGCGATAAGTCGTGTCTTACCGGGTTGGACTCAAGACGATAGTTACCG
GATAAGGCGCAGCGGTCGGGCTGAACGGGGGGTTCGTGCACACAGCCCAGCTTGGAG
CGAACGACCTACACCGAACTGAGATACCTACAGCGTGAGCTATGAGAAAGCGCCACG
CTTCCCGAAGGGAGAAAGGCGGACAGGTATCCGGTAAGCGGCAGGGTCGGAACAGGA
GAGCGCACGAGGGAGCTTCCAGGGGGAAACGCCTGGTATCTTTATAGTCCTGTCGGG
TTTCGCCACCTCTGACTTGAGCGTCGATTTTTGTGATGCTCGTCAGGGGGGCGGAGC
CTATGGAAAAACGCCAGCAACGCGGCCTTTTTACGGTTCCTGGCCTTTTGCTGGCCT
TTTGCTCACATGT
SEQ ID NO: 48
   Primer Ins1
   GTCTCCCAGATCACTGTCCTTC
SEQ ID NO: 49
   Primer Ins2r
   TCTTCCCCATCTCCTGACTATG
SEQ ID NO: 50
   Primer HiBiTR
   AATCTTCTTGAACAGCCGCCAG
SEQ ID NO: 51
   HA tag
   YPYDVPDYA
SEQ ID NO: 52
   FLAG tag
   DYKDDDDK
SEQ ID NO: 53
   Primer pEB-Ins F
   CCTCACTAAAGGGGTACCATGGCCCTGTGGATGCGCCTCCTGCC
SEQ ID NO: 54
   Primer pEB-Ins R
   AAGCTTATCGATACCGTCTCACTAGTTGCAGTAGTTCTCCAGCTGGTAG
SEQ ID NO: 55
   hIns-encoding gene 
ATGGCCCTGTGGATGCGCCTCCTGCCCCTGCTGGCGCTGCTGGCCCTCTGGGGACCT

GACCCAGCCGCAGCCTTTGTGAACCAACACCTGTGCGGCTCACACCTGGTGGAAGCT

CTCTACCTAGTGTGCGGGGAACGAGGCTTCTTCTACACACCCAAGACCCGCCGGGAG

GCAGAGGACCTGCAGGTGGGGCAGGTGGAGCTGGGCGGGGGCCCTGGTGCAGGCAGC

CTGCAGCCCTTGGCCCTGGAGGGGTCCCTGCAGAAGCGTGGCATTGTGGAACAATGC

TGTACCAGCATCTGCTCCCTCTACCAGCTGGAGAACTACTGCAACTAG
SEQ ID NO: 56
   hIns amino acid sequence
SEQ ID NO: 57
   Primer Ins-HA R
   CTGGAACATCGTATGGGTACACCTGCAGGTCCTCTGCCTCCC
SEQ ID NO: 58
   Primer HA-Ins F
   CCATACGATGTTCCAGATTACGCTGGCGGGGGCCCTGGTGCAGGCAG
SEQ ID NO: 59
   hIns-HiBiT5-HA-encoding gene 
ATGGCCCTGTGGATGCGCCTCCTGCCCCTGCTGGCGCTGCTGGCCCTCTGGGGACCT

GACCCAGCCGCAGCCTTTGTGAACCAACACCTGTGCGGCTCACACCTGGTGGAAGCT

CTCTACCTAGTGTGCGGGGAACGAGGCTTCTTCTACACACCCAAGACCCGCCGGGAG

GCAGAGGACCTGCAGGTGTACCCATACGATGTTCCAGATTACGCTGGCGGGGGCCCT

GGTGCAGGCAGCCTGCAGCCCTTGGCCCTGGAGGGGTCCCTGCAGAAGCGTGGCATT

GTGGAACAATGCTGTACCAGCATCTGCTCCCTCTACCAGCTGGAGAACTACTGCAAC

TAA
SEQ ID NO: 60
   hIns-HiBiT5-HA amino acid sequence 
MALWMRLLPLLALLALWGPDPAAAFVNQHLCGSHLVEALYLVCGERGFFYTPKTRRE

AEDLQVYPYDVPDYAGGGPGAGSLQPLALEGSLQKRGIVEQCCTSICSLYQLENYCN
SEQ ID NO: 61
   Primer Ins-FlagR
   CGTCGTCATCCTTGTAATCCACCTGCAGGTCCTCTGCCTCCC
SEQ ID NO: 62
   Primer Flag-InsF
   ACAAGGATGACGACGATAAGGGCGGGGGCCCTGGTGCAGGCAG
SEQ ID NO: 63
   hIns-HiBiT5-FLAG-encoding gene 
ATGGCCCTGTGGATGCGCCTCCTGCCCCTGCTGGCGCTGCTGGCCCTCTGGGGACCT

GACCCAGCCGCAGCCTTTGTGAACCAACACCTGTGCGGCTCACACCTGGTGGAAGCT

CTCTACCTAGTGTGCGGGGAACGAGGCTTCTTCTACACACCCAAGACCCGCCGGGAG

GCAGAGGACCTGCAGGTGGATTACAAGGATGACGACGATAAGGGCGGGGGCCCTGGT

GCAGGCAGCCTGCAGCCCTTGGCCCTGGAGGGGTCCCTGCAGAAGCGTGGCATTGTG

GAACAATGCTGTACCAGCATCTGCTCCCTCTACCAGCTGGAGAACTACTGCAACTAA

GAATTCA
SEQ ID NO: 64
   hIns-HiBiT5-FLAG amino acid sequence 
MALWMRLLPLLALLALWGPDPAAAFVNQHLCGSHLVEALYLVCGERGFFYTPKTRRE

AEDLQVDYKDDDDKGGGPGAGSLQPLALEGSLQKRGIVEQCCTSICSLYQLENYCN
SEQ ID NO: 65
   hUcn3
   Primer hUcn3 F
   CCTCACTAAAGGGGTACCATGCTGATGCCGGTCCACTTCCTGC
SEQ ID NO: 66
   Primer hUcn3 R
   AAGCTTATCGATACCGTCCTACTTCTTCCTCCCAATTTGCGCCATCAG
SEQ ID NO: 67
   hUcn3-encoding gene 
ATGCTGATGCCGGTCCACTTCCTGCTGCTCCTGCTGCTGCTCCTGGGGGGCCCCAGG

ACAGGCCTCCCCCACAAGTTCTACAAAGCCAAGCCCATCTTCAGCTGCCTCAACACC

GCCCTGTCTGAGGCTGAGAAGGGCCAGTGGGAGGATGCATCCCTGCTGAGCAAGAGG

AGCTTCCACTACCTGCGCAGCAGAGACGCCTCTTCGGGAGAGGAGGAGGGCAAA

GAGAAAAAGACTTTCCCCATCTCTGGGGCCAGGGGTGGAGCCAGAGGCACCCGGTAC

AGATACGTGTCCCAAGCACAGCCCAGGGGAAAGCCACGCCAGGACACGGCCAAGAGT

CCCCACCGCACCAAGTTCACCCTGTCCCTCGACGTCCCCACCAACATCATGAACCTC

CTCTTCAACATCGCCAAGGCCAAGAACCTGCGTGCCCAGGCGGCCGCCAATGCCCAC

CTGATGGCGCAAATTGGGAGGAAGAAGTAG
SEQ ID NO: 68
   hUcn3 amino acid sequence 
MLMPVHFLLLLLLLLGGPRTGLPHKFYKAKPIFSCLNTALSEAEKGQWEDASLLSKR

SFHYLRSRDASSGEEEEGKEKKTFPISGARGGARGTRYRYVSQAQPRGKPRQDTAKS

PHRTKFTLSLDVPTNIMNLLFNIAKAKNLRAQAAANAHLMAQIGRKK
SEQ ID NO: 69
   hKLF9
   Primer hKLF9 F
   CCTCACTAAAGGGGTACCATGTCCGCGGCCGCCTACATGGACTTCG
SEQ ID NO: 70
   Primer hKLF9 R
   AAGCTTATCGATACCGTCTCACAAAGCGTTGGCCAGCGCCTTTTTCGATC
SEQ ID NO: 71
   hKLF9-encoding gene 
ATGTCCGCGGCCGCCTACATGGACTTCGTGGCTGCCCAGTGTCTGGTTTCCATTTCG

AACCGCGCTGCGGTGCCGGAGCATGGGGTCGCTCCGGACGCCGAGCGGCTGCGACTA

CCTGAGCGCGAGGTGACCAAGGAGCACGGTGACCCGGGGGACACCTGGAAGGATTAC

TGCACACTGGTCACCATCGCCAAGAGCTTGTTGGACCTGAACAAGTACCGACCCATC

CAGACCCCCTCCGTGTGCAGCGACAGTCTGGAAAGTCCAGATGAGGATATGGGATCC

GACAGCGACGTGACCACCGAATCTGGGTCGAGTCCTTCCCACAGCCCGGAGGAGAGA

CAGGATCCTGGCAGCGCGCCCAGCCCGCTCTCCCTCCTCCATCCTGGAGTGGCTGCG

AAGGGGAAACACGCCTCCGAAAAGAGGCACAAGTGCCCCTACAGTGGCTGTGGGAAA

GTCTATGGAAAATCCTCCCATCTCAAAGCCCATTACAGAGTGCATACAGGTGAACGG

CCCTTTCCCTGCACGTGGCCAGACTGCCTTAAAAAGTTCTCCCGCTCAGACGAGCTG

ACCCGCCACTACCGGACCCACACTGGGGAAAAGCAGTTCCGCTGTCCGCTGTGTGAG

AAGCGCTTCATGAGGAGTGACCACCTCACAAAGCACGCCCGGCGGCACACCGAGTTC

CACCCCAGCATGATCAAGCGATCGAAAAAGGCGCTGGCCAACGCTTTGTGA
SEQ ID NO: 72
   hKLF9 amino acid sequence

MSAAAYMDFVAAQCLVSISNRAAVPEHGVAPDAERLRLPEREVTKEHGDPGDTWKDY

CTLVTIAKSLLDLNKYRPIQTPSVCSDSLESPDEDMGSDSDVTTESGSSPSHSPEER

QDPGSAPSPLSLLHPGVAAKGKHASEKRHKCPYSGCGKVYGKSSHLKAHYRVHTGER

PFPCTWPDCLKKFSRSDELTRHYRTHTGEKQFRCPLCEKRFMRSDHLTKHARRHTEF

HPSMIKRSKKALANAL

## Claims

1. A method for quantifying insulin secretion capacity in a human cell, comprising culturing a recombinant cell and measuring an amount of a secreted fused C-peptide, the recombinant cell being prepared by replacing an insulin gene in the genome of a human cultured cell line with a gene which expresses a fused C-peptide having a detectable tag peptide inserted in a C-peptide chain in a human proinsulin molecule.

2. The quantification method according to claim 1, wherein an insertion position of the detectable tag peptide is a position corresponding to the C-terminal side of 7V of the C-peptide chain.

3. The quantification method according to claim 1 or 2, wherein the detectable tag peptide is a tag peptide selected from the group consisting of a tag peptide detectable by chemiluminescent assay, and a tag peptide detectable by immunoassay.

4. The quantification method according to any one of claims 1 to 3, wherein the detectable tag peptide is a tag peptide selected from the group consisting of a tag peptide capable of binding to luciferase, a DDDDK-containing tag peptide, and a hemagglutinin-derived tag peptide.

5. The quantification method according to any one of claims 1 to 4, wherein the detectable tag peptide is a tag peptide capable of binding to luciferase.

6. The quantification method according to any of [1] to [5], wherein the detectable tag peptide is a tag peptide selected from the group consisting of VSGWRLFKKIS (SEQ ID NO: 1), YPYDVPDYA (SEQ ID NO: 51), and YPYDVPDYA (SEQ ID NO: 51).

7. The quantification method according to any one of claims 1 to 6, wherein the human cultured cell line is (1) a human cultured cell line, (2) a human cell having 3 genes Ngn3, Mafa, and Pdx1 introduced therein, or (3) a human cell having 3 genes Ngn3, Mafa, and Pdx1; KLF9, KLF11, or KLF12 gene; and UCN3 gene simultaneously introduced therein.

8. A recombinant cell prepared by replacing an insulin gene in the genome of a human cultured cell line with a gene which expresses a fused C-peptide having a detectable tag peptide inserted in a C-peptide chain in a human proinsulin molecule.

9. The recombinant cell according to claim 8, wherein an insertion position of the detectable tag peptide is a position corresponding to the C-terminal side of 7V of the C-peptide chain.

10. The recombinant cell according to claim 8 or 9, wherein the detectable tag peptide is a tag peptide selected from the group consisting of a tag peptide detectable by chemiluminescent assay, and a tag peptide detectable by immunoassay.

11. The recombinant cell according to any one of claims 8 to 10, wherein the detectable tag peptide is a tag peptide selected from the group consisting of a tag peptide capable of binding to luciferase, a DDDDK-containing tag peptide, and a hemagglutinin-derived tag peptide.

12. The recombinant cell according to any one of claims 8 to 11, wherein the detectable tag peptide is a tag peptide capable of binding to luciferase.

13. The recombinant cell according to any one of claims 8 to 12, wherein the detectable tag peptide is a tag peptide selected from the group consisting of VSGWRLFKKIS (SEQ ID NO: 1), YPYDVPDYA (SEQ ID NO: 51), and YPYDVPDYA (SEQ ID NO: 51).

14. The recombinant cell according to any one of claims 8 to 13, wherein the human cultured cell line is (1) a human cultured cell line, (2) a human cell having 3 genes Ngn3, Mafa, and Pdx1 introduced therein, or (3) a human cell having 3 genes Ngn3, Mafa, and Pdx1; KLF9, KLF11, or KLF12 gene; and UCN3 gene simultaneously introduced therein.

15. A method for screening for a substance that promotes human insulin production and secretion, comprising culturing the recombinant cell according to any one of claims 8 to 14 in the presence of a test substance, and measuring an amount of a secreted fused C-peptide.

16. A human insulin production and secretion promoter, comprising a substance obtained by a screening method according to claim 15.

17. An insulin production and secretion promoter, comprising one or two members selected from the group consisting of valproic acid and zebularine.
